# EUROPEAN PATENT APPLICATION

(11) **EP 3 907 227 A1**
(43) Date of publication of application: **10.11.2021**
(21) Application number: 20305456.4
(22) Date of filing: 07.05.2020
(51) Int. Cl.: C07D 491/22, A61K 31/4741, A61P 33/06

(54) **TRILOBINE DERIVATIVES AND THEIR USE THEREOF IN THE TREATMENT OF MALARIA**

(71) Applicant: Institut Pasteur, 75015 Paris (FR); Centre National de la Recherche Scientifique, 75016 Paris (FR); INSTITUT NATIONAL DE LA SANTE ET DE LA RECHERCHE MEDICALE - INSERM, 75013 Paris (FR)
(72) Inventor: NARDELLA, Flore, 75015 Paris (FR); HALBY, Ludovic, 94700 Maisons-Alfort (FR); HAITHAM, Hassam, 91330 Yerres (FR); MENARD, Didier, 44000 Nantes (FR); SCHERF, Artur, 75015 Paris (FR); ARIMONDO, Paola B., 75013 Paris (FR)
(74) Representative: Gevers & Orès

(57) **Abstract**

The present invention is in the field of therapeutic drugs to treat malaria. In particular, the invention provides trilobine derivatives *per se,* and trilobine derivatives for use in the treatment of malaria, for example drug-resistant malaria.

## Description

The present invention is in the field of therapeutic drugs to treat malaria. In particular, the invention provides trilobine derivatives *per se,* and trilobine derivatives for use in the treatment of malaria, for example drug-resistant malaria.

Malaria is a devastating infectious disease which affect each year more than 200 million people and claimed 435,000 deaths worldwide in 2018. As a major health concern in Africa, Asia, the Middle East, and Central and South America, about 40% of the world's population live in areas where malaria is transmitted. Approximately 90% of both cases and deaths occurred in Africa, mostly among children, 78% of reported deaths belonging to children below 5 years age.

Malaria is caused by blood protozoa of the genus Plasmodium, transmitted by Anopheles spp., and shows symptoms such as intermittent paroxysm of fever, anemia, splenomegaly, etc. The five known species of *Plasmodium genus* that causes malaria in human are *Plasmodium falciparum, Plasmodium vivax, Plasmodium malariae, Plasmodium ovale,* and *Plasmodium knowlesi.* Among them, *P. falciparum* is the most virulent parasite, with high mortality and morbidity rate, causing the most severe symptoms: it progresses to severe malaria easily at 1 or 2 days after the onset of disease, accompanied with coma and multiple organ failure that eventually leads to the death of the patients.

The discovery of the synthetic drug chloroquine (CQ, first of the quinoline class) in the 1940s helped to treat and prevent malaria throughout the world in 1950s. However, the therapeutic efficacy of CQ and efforts to eradicate malaria worldwide were diminished due to the occurrence of CQ resistance. The failure of these eradication programs led to re-emergence of malaria and spread of CQ-resistant parasite from Southeast Asia and South America to Africa. Due to the lack of potent and affordable drug for malaria treatment, the spread of CQ-resistant parasite to Africa around 1980s claimed 2-3-fold increase in malaria-related deaths. Hence, CQ was replaced with sulfadoxine/pyrimethamine (SP, of the antifolate class) as the first-line of treatment for malaria; however, parasite became resistant to SP and spread widely. Use of antimalarial drugs as combination therapy instead of monotherapy has been in practice to increase the efficacy of drug and to delay the emergence of drug resistance parasite. Since 2001, artemisinin-based combination therapies (ACTs) has been most widely and effectively used for malaria treatment. Recently, resistance to ACTs (Artesunate-Mefloquine and Dihydroartemisinin-Piperaquine) has been reported in Southeast Asia (Greater Mekong Subregion, GMS) increasing the global alarm for malaria treatment and control.

The risk of ACT-resistant parasites spreading from the Greater Mekong Subregion to Africa is extremely worrisome, as it happened previously with chloroquine- and sulfadoxine/pyrimethamine-resistant parasites.

Drug resistant malaria is thus a serious clinical and public health problem. Therefore, novel pharmaceutical compositions which kill drug-resistant malaria parasites, are needed for successful therapy.

The present invention thus concerns a compound of following formula (I) 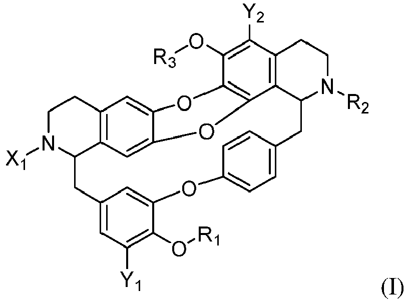 Wherein,
Y₁ and Y₂ are each independently H or (C₁-C₁₀)-alkyl, in which the fragments (C₁-C₁₀)-alkyl are optionally substituted by at least one group selected from NR_{Y1}R_{Y2};
R_{Y1} and R_{Y2} are each independently H or (C₁-C₁₀)-alkyl;
X₁ is H, methyl, ethyl, (C₁-C₁₀)-alkyl-R₄, (C₂-C₁₀)-alkenyl, (C₂-C₁₀)-alkenyl-R₄, CONHR₅, (CH₂-CH₂O)ₘ-H with m=2-4, in which the fragments (C₁-C₁₀)-alkyl are optionally substituted by at least one group selected from halogens;
and N-X₁ optionally represents N⁺-O⁻;
R₂ is chosen from H, methyl, ethyl, (C₁-C₁₀)-alkyl-R₄-, (C₂-C₁₀)-alkenyl, (C₂-C₁₀)-alkenyl-R_{4'}, CONHR_{5'}, (CH₂-CH₂O)ₘ-H with m=2-4, (C₁-C₁₀)-alkyl substituted by at least one group selected from halogen, -OH and -O-(C₁-C₁₀)-alkyl;
R₄ is OH, O-(C₁-C₁₀)-alkyl, O-(C₅-C₁₀)-aryl, NO₂, CN, (3-12)-membered-heterocycle, O-((CH₂)₂O)ₙ-H with n=1-3, CONR_{c}R_{d}, halogen, COOR_{c}, CF₃, or (C₃-C₁₂)-cycloalkyl, in which (C₅-C₁₀)-aryl and (3-12)-membered-heterocycle are optionally substituted by at least one group selected from NR_{c}R_{d}, ORₑ, (C₁-C₁₀)-alkyl, halogen and oxo (=O);
R₅ is H, or a group chosen among (C₁-C₁₀)-alkyl-R₆, (C₃-C₁₂)-cycloalkyl, (C₂-C₁₀)-alkenyl, and a (3-12)-membered-heteroaryl, said group being optionally substituted by at least one group selected from halogen, CN, NR_{c}R_{d}, NO₂, CONR_{c}R_{d}, COOR_{c}, CF₃, ORₑ,
R_{4'} is OH, O-(C₁-C₁₀)-alkyl, O-(C₅-C₁₀)-aryl, NO₂, CN, NR_{c}R_{d}, (3-12)-membered-heterocycle, O-((CH₂)₂O)ₙ-H with n=1-3, CONR_{c}R_{d}, halogen, COOR_{c}, CF3, or (C₃-C₁₂)-cycloalkyl, in which (C₅-C₁₀)-aryl and (3-12)-membered-heterocycle are optionally substituted by at least one group selected from NR_{c}R_{d}, OR_{c}, (C₁-C₁₀)-alkyl, halogen and oxo (=O);
R_{5'} is H, or a group chosen among (C₁-C₁₀)-alkyl-R_{6'}, (C₃-C₁₂)-cycloalkyl, (C₂-C₁₀)-alkenyl, benzyl, and, only when R₁ and X₁ are not H, Me, said groups being optionally substituted by at least one group selected from halogen, CN, NR_{c}R_{d}, NO₂, CONR_{c}R_{d}, COOR_{c}, CF₃, ORₑ;
R6 is NR_{c}R_{d}, (3-12)-membered-heteroaryl or (3-12)-membered-heterocycle, in which the fragment (3-12)-membered-heteroaryl or (3-12)-membered-heterocycle is optionally substituted by at least one group selected from NR_{c}R_{d}, ORₑ, (C₁-C₁₀)-alkyl, (C₁-C₁₀)-alkyl-(3-12)-membered-heterocycle, halogen and oxo;
R_{6'} is NR_{c}R_{d} or (3-12)-membered-heterocycle, in which the (3-12)-membered-heterocycle is optionally substituted by at least one group selected from (C₁-C₁₀)-alkyl;
R₁ is H, methyl, -(C₁-C₆) alkyl-NRaRb, COR₇, (3-12)-membered-azaheterocycle optionally substituted by at least one group selected from (C₁-C₁₀)-alkyl, or (C₁-C₆)-alkyl substituted by at least one group selected from epoxide and (3-12)-membered-azaheterocycle, both optionally substituted by at least one group selected from (C₁-C₁₀)-alkyl,
Rₐ and R_{b} are independently from each other (C₁-C₆) alkyl or H, at least one of Ra and R_{b} being a (C₁-C₆) alkyl;
R_{c}, R_{d} and Rₑ are for each occurence independently from each other (C₁-C₆) alkyl or H; R₃ is H, (C₁-C₆) alkyl, (C₁-C₆) alkyl substituted by NR_{c}R_{d};
R₇ is (C₁-C₁₀)-alkyl, (C₅-C₁₀)-aryl;
with the proviso that:
- in case Y₁ and Y₂ are H, and R₁, R₂, R₃ and X₁ are independently chosen from H and methyl, then R₃ is H and R₂ is H;
   or a pharmaceutically acceptable salt or solvate thereof,
   for use in the treatment and/or prevention of malaria.

For the purpose of the invention, the term "pharmaceutically acceptable" is intended to mean what is useful to the preparation of a pharmaceutical composition, and what is generally safe and non-toxic, for a pharmaceutical use.

The term "pharmaceutically acceptable salt or solvate" is intended to mean, in the framework of the present invention, a salt or solvate of a compound which is pharmaceutically acceptable, as defined above, and which possesses the pharmacological activity of the corresponding compound.

The pharmaceutically acceptable salts comprise:
(1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and
(2) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

Acceptable solvates for the therapeutic use of the compounds of the present invention include conventional solvates such as those formed during the last step of the preparation of the compounds of the invention due to the presence of solvents. As an example, mention may be made of solvates due to the presence of water (these solvates are also called hydrates) or ethanol.

It is recognized that compounds of the present invention may exist in various stereoisomeric forms. As such, the compounds of the present invention include both diastereomers and enantiomers. The compounds are normally prepared as racemates and can conveniently be used as such, but individual enantiomers can be isolated or synthesized by conventional techniques if so desired. Such racemates and individual enantiomers and mixtures thereof form part of the present invention.

It is well known in the art how to prepare and isolate such optically active forms. Specific stereoisomers can be prepared by stereospecific synthesis using enantiomerically pure or enantiomerically enriched starting materials. The specific stereoisomers of either starting materials or products can be resolved and recovered by techniques known in the art, such as resolution of racemic forms, normal, reverse-phase, and chiral chromatography, recrystallization, enzymatic resolution, or fractional recrystallization of addition salts formed by reagents used for that purpose. Useful methods of resolving and recovering specific stereoisomers described in Eliel, E. L.; Wilen, S.H. Stereochemistry of Organic Compounds; Wiley: New York, 1994, and Jacques, J, et al. Enantiomers, Racemates, and Resolutions; Wiley: New York, 1981, each incorporated by reference herein in their entireties.

About the two following asymmetric carbon atoms: 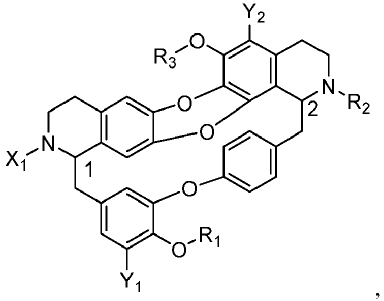 compounds of the invention are for example (1S, 2S), (1R, 2R), (1R, 2S), or (1S, 2R), and in particular (1S, 2S).

The term "(C₁-C₆)alkyl", as used in the present invention, refers to a straight or branched saturated hydrocarbon chain containing from 1 to 6 carbon atoms including, but not limited to, methyl, ethyl, n-propyl, iso-propyl, n-butyl, iso-butyl, sec-butyl, t-butyl, n-pentyl, n-hexyl, and the like.

The term "(C₂-C₆)alkenyl", as used in the present invention, refers to a straight or branched unsaturated hydrocarbon chain containing from 2 to 6 carbon atoms and comprising at least one double bond, notably one double bond, including, but not limited to, ethenyl, propenyl, butenyl, pentenyl, hexenyl and the like. It can be in particular an allyl group.

The term "aryl", as used in the present invention, refers to an aromatic hydrocarbon group comprising preferably 6 to 10 carbon atoms and comprising one or more, notably 1 or 2, fused rings, such as, for example, a phenyl or naphtyl group. Advantageously, it will be a phenyl group.

The term "aryl-(C₁-C₆)alkyl", as used in the present invention, refers to an aryl group as defined above bound to the molecule via a (C₁-C₆)alkyl group as defined above. In particular, the aryl-(C₁-C₆)alkyl group is a benzyl group.

The term "(C₁-C₆)alkyl-aryl", as used in the present invention, refers to a (C₁-C₆)alkyl group as defined above bound to the molecule via an aryl group as defined above. In particular, it can be a tolyl group (-Ph-CH₃).

The term "heterocycle" as used in the present invention refers to a saturated, unsaturated or aromatic hydrocarbon monocycle or polycycle (comprising fused, bridged or spiro rings), such as a bicycle, in which one or more, advantageously 1 to 4, and more advantageously 1 or 2, carbon atoms have each been replaced with a heteroatom selected from nitrogen, oxygen and sulphur atoms, and notably being a nitrogen atom. Advantageously, the heterocycle comprises 5 to 15, notably 5 to 10 atoms in the ring(s). The ring(s) of the heterocycle has/have advantageously 5 or 6 members.

According to a particular embodiment, the heterocycle is a saturated, unsaturated or aromatic hydrocarbon monocycle or bicycle (comprising fused, bridged or spiro rings, notably fused rings), each cycle having 5 or 6 members and 1 to 4, notably 1 or 2, carbon atoms having each been replaced with a nitrogen or oxygen atom, notably a nitrogen atom.

A heterocycle can be notably thiophene, furan, pyrrole, imidazole, pyrazole, oxazole, isoxazole, thiazole, isothiazole, triazoles (1,2,3-triazole and 1,2,4-triazole), benzofuran, indole, benzothiophene, benzimidazole, indazole, benzoxazole, benzisoxazole, benzothiazole, benzisothiazole, pyridine, pyrimidine, pyridazine, pyrazine, triazine, quinoline, isoquinoline, quinoxaline, quinazoline, piperidine, piperazine, triazinane, morpholine, pyrrolidine, azepane, dihydropyridines, dihydropyrimidines (notably 1,2- dihydropyrimidine), dihydropyridazines, dihydropyrazines, dihydrotriazines, tetrahydropyridines, tetrahydropyrimidines, tetrahydropyridazines, tetrahydropyrazines, tetrahydrotriazines, etc.

A heterocycle can also be notably tetrahydrofuran, oxetane, or oxirane.

The term "heterocycle-(C₁-C₆)alkyl", as used in the present invention, refers to a heterocycle group as defined above bound to the molecule via a (C₁-C₆)alkyl group as defined above.

The term "heteroaryl" as used in the present invention refers to an aromatic heterocycle as defined above.

According to a particular embodiment, the heteroaryl is an aromatic hydrocarbon monocycle or bicycle (i.e. comprising fused rings), each cycle having 5 or 6 members, notably 6 members, and 1 to 4, notably 1 or 2, carbon atoms having each been replaced with a nitrogen or oxygen atom, notably a nitrogen atom.

A heteroaryl can be notably thiophene, furan, pyrrole, imidazole, pyrazole, oxazole, isoxazole, thiazole, isothiazole, triazoles (1,2,3-triazole and 1,2,4-triazole), benzofuran, indole, benzothiophene, benzimidazole, indazole, benzoxazole, benzisoxazole, benzothiazole, benzisothiazole, pyridine, pyrimidine, pyridazine, pyrazine, triazine, quinoline, isoquinoline, quinoxaline, quinazoline, etc.

The term "heteroaryl-(C₁-C₆)alkyl", as used in the present invention, refers to a heteroaryl group as defined above bound to the molecule via a (C₁-C₆)alkyl group as defined above.

The term "halogen", as used in the present invention, refers to a fluorine, bromine, chlorine or iodine atom.

In a particular embodiment, Y₁ and/or Y₂, in particular Y₁ and Y₂, are H.

In a particular embodiment, R₃ is H or methyl, in particular methyl.

In a particular embodiment, R₃ is H, R₁ being in particular H.

In a particular embodiment, Y₁ and Y₂, are H, and R₃ is methyl.

In a particular embodiment, at least one of R₁, R₂ and X₁ is not H nor methyl.

In a particular embodiment, one of R₁, R₂ and X₁ is not H nor methyl, the two others being H or methyl.

In a particular embodiment, one of R₁, R₂ and X₁ is not H nor methyl, the two others being methyl.

In a particular embodiment, R₁ is not H nor methyl, R₂ and X₁ being in particular independently H or methyl.

In a particular embodiment, R₁ is not H nor methyl, R₂ and X₁ being methyl.

In a particular embodiment, R₂ is not H nor methyl, R₁ and X₁ being in particular independently H or methyl.

In a particular embodiment, R₂ is not H nor methyl, R₁ and X₁ being methyl.

In a particular embodiment, X₁ is not H nor methyl, R₁ and R₂ being in particular independently H or methyl.

In a particular embodiment, X₁ is not H nor methyl, R₁ and R₂ being methyl.

In a particular embodiment, X₁ and R₂ are not H nor methyl, X₁ and R₂ being in particular identical, R₁ being notably H or methyl.

In a particular embodiment, X₁ and R₂ are not H nor methyl, X₁ and R₂ being in particular identical, R₁ being methyl.

In a particular embodiment, R₁, R₂, R₃ and X₁ are independently chosen from H and methyl, with R₃ being H, R₁ being in particular H.

In a particular embodiment, R₁, R₂, R₃ and X₁ are independently chosen from H and methyl, with R₃ being H, R₁ and R₂ being in particular H.

In a particular embodiment, X₁ is H, methyl, CONHR₅, or (CH₂-CH₂O)ₘ-H with m=2-4.

In a particular embodiment, R₅ is a group chosen among (C₁-C₁₀)-alkyl-R₆ and (3-12)-membered-heteroaryl, in particular pyridyl.

In a particular embodiment, R₆ is NR_{c}R_{d}, (3-12)-membered-heteroaryl, in particular pyridyl, or (3-12)-membered-heterocycle, in particular piperazinyl, in which the fragment (3-12)-membered-heteroaryl or (3-12)-membered-heterocycle is optionally substituted by at least one group selected from (C₁-C₁₀)-alkyl;

In a particular embodiment R₂ is chosen from H, methyl, (C₁-C₁₀)-alkyl-R_{4'}, CONHR_{5'}, and (CH₂-CH₂O)ₘ-H with m=2-4, (C₁-C₁₀)-alkyl being optionally substituted by at least one -OH.

In a particular embodiment, R_{4'} is NR_{c}R_{d}.

In a particular embodiment, R_{5'} is a group chosen among (C₁-C₁₀)-alkyl-R_{6'}, benzyl, and, only when R₁ and X₁ are not H, Me.

In a particular embodiment, R_{6'} is NR_{c}R_{d}.

In a particular embodiment, R₇ is (C₁-C₁₀)-alkyl, (C₅-C₁₀)-aryl.

In a particular embodiment, the compound as defined above is of following formula (II) 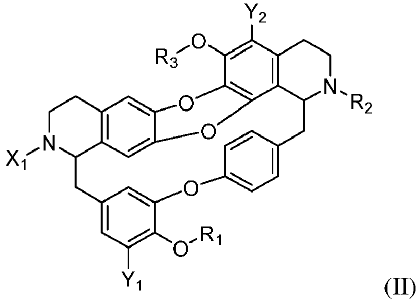 Wherein:
Y₁, Y₂ and R₃ are as defined above, Y₁ and Y₂, being in particular H, and R₃ being in particular methyl,
X₁ is H, methyl, CONHR₅, (CH₂-CH₂O)ₘ-H with m=2-4;
R₂ is chosen from H, methyl, (C₁-C₁₀)-alkyl-R₄-, CONHR_{5'}, and (CH₂-CH₂O)ₘ-H with m=2-4, (C₁-C₁₀)-alkyl being optionally substituted by at least one -OH;
R₅ is a group chosen among (C₁-C₁₀)-alkyl-R₆, and (3-12)-membered-heteroaryl, in particular pyridyl;
R_{4'} is NR_{c}R_{d};
R_{5'} is a group chosen among (C₁-C₁₀)-alkyl-R_{6'}, benzyl, and, only when R₁ and X₁ are not H, Me;
R₆ is NR_{c}R_{d}, (3-12)-membered-heteroaryl, in particular pyridyl, or (3-12)-membered-heterocycle, in particular piperazinyl, in which the fragment (3-12)-membered-heteroaryl or (3-12)-membered-heterocycle is optionally substituted by at least one group selected from (C₁-C₁₀)-alkyl;
R_{6'} is NR_{c}R_{d};
R₁ is H, methyl, -(C₁-C₆)alkyl-NRₐR_{b}, COR₇, (3-12)-membered-azaheterocycle, in particular azepanyl, optionally substituted by at least one group selected from (C₁-C₁₀)-alkyl, or (C₁-C₆)-alkyl substituted by at least one group selected from epoxide and (3-12)-membered-azaheterocycle, in particular pyrrolidinyl or piperazinyl, both epoxide and (3-12)-membered-azaheterocycle being optionally substituted by at least one group selected from (C₁-C₁₀)-alkyl,
Rₐ and R_{b} are independently from each other (C₁-C₆) alkyl or H, at least one of Ra and R_{b} being a (C₁-C₆) alkyl;
R_{c}, R_{d} and Rₑ are for each occurence independently from each other (C₁-C₆) alkyl or H;
R₇ is (C₁-C₁₀)-alkyl, (C₅-C₁₀)-aryl, in particular (C₁-C₁₀)-alkyl;
with the proviso that:
- in case Y₁ and Y₂ are H, and R₁, R₂, R₃ and X₁ are independently chosen from H and methyl, then R₃ is H and R₂ is H.

In a particular embodiment, the compound as defined above is of following formula (IIa) 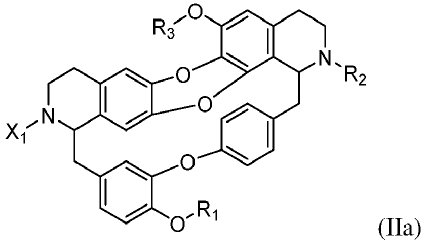 Wherein:
R3 is as defined above, R₃ being in particular H or methyl;
X₁ is H, methyl, CONHR₅;
R₂ is chosen from H, methyl, (C₁-C₁₀)-alkyl-R_{4'}, (C₁-C₁₀)-alkyl being optionally substituted by at least one or two -OH;
R₅ is a group chosen among (C₁-C₁₀)-alkyl-R₆;
R_{4'} is NR_{c}R_{d};
R₆ is (3-12)-membered-heteroaryl, in particular pyridyl, , in which the fragment (3-12)-membered-heteroaryl is optionally substituted by at least one group selected from (C₁-C₁₀)-alkyl;
R₁ is H, methyl, -(C₁-C₆)alkyl-NRₐR_{b}, in particular -(C₃)alkyl-NRₐR_{b}, or (C₁-C₆)-alkyl substituted by at least one group selected from (3-12)-membered-azaheterocycle, in particular pyrrolidinyl or piperazinyl, said (3-12)-membered-azaheterocycle being optionally substituted by at least one group selected from (C₁-C₁₀)-alkyl,
Rₐ and R_{b} are independently from each other (C₁-C₆) alkyl or H, at least one of Rₐ and R_{b} being a (C₁-C₆) alkyl;
R_{c}, R_{d} and Rₑ are for each occurence independently from each other (C₁-C₆) alkyl or H;
with the proviso that:
- in case R₁, R₂, R₃ and X₁ are independently chosen from H and methyl, then R₃ is H, R₂ is H, and X₁ is H.

In a particular embodiment, the compound as defined above is of following formula (IIa-1) 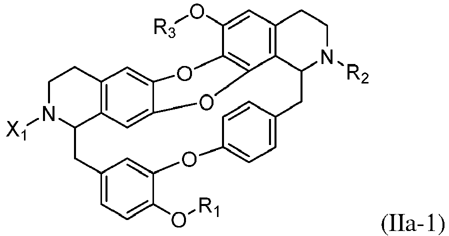 Wherein:
R₃ is methyl;
X₁ is methyl, CONHR₅;
R₂ is chosen from methyl, (C₁-C₁₀)-alkyl-R_{4'}, (C₁-C₁₀)-alkyl being optionally substituted by at least one or two -OH;
R₅ is a group chosen among (C₁-C₁₀)-alkyl-R₆;
R_{4'} is NR_{c}R_{d};
R₆ is (3-12)-membered-heteroaryl, in particular pyridyl, in which the fragment (3-12)-membered-heteroaryl is optionally substituted by at least one group selected from (C₁-C₁₀)-alkyl;
R₁ is methyl, -(C₁-C₆)alkyl-NRₐR_{b}, in particular -(C₃)alkyl-NRₐR_{b}, or (C₁-C₆)-alkyl substituted by at least one group selected from (3-12)-membered-azaheterocycle, in particular pyrrolidinyl or piperazinyl, said (3-12)-membered-azaheterocycle being optionally substituted by at least one group selected from (C₁-C₁₀)-alkyl,
Rₐ and R_{b} are independently from each other (C₁-C₆) alkyl or H, at least one of Ra and R_{b} being a (C₁-C₆) alkyl;
R_{c}, R_{d} and Rₑ are for each occurence independently from each other (C₁-C₆) alkyl or H; with the proviso that R₁, R₂, R₃ and X₁ are not all independently chosen from H and methyl.

In a particular embodiment, the compound as defined above is of following formula (IIa-2) 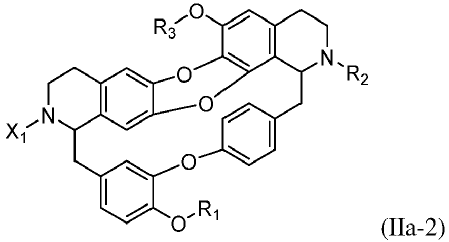 Wherein:
- R₁: is H, methyl, or -(C₁-C₆)alkyl-NRₐR_{b}, in particular -(C₃)alkyl-NRₐR_{b};
- R₃: is H;
- X₁: is H or methyl;
- R₂: is H or methyl;
and in particular wherein:
- R₁: is H;
- R₃: is H;
- X₁: is H or methyl;
- R₂: is H.

In a particular embodiment, in case R₁, R₂, R₃ and X₁ are independently chosen from H and methyl, then R₁ and R₃ are H, and R₂ is H.

In a particular embodiment, the compound as defined above is selected from the following compounds:

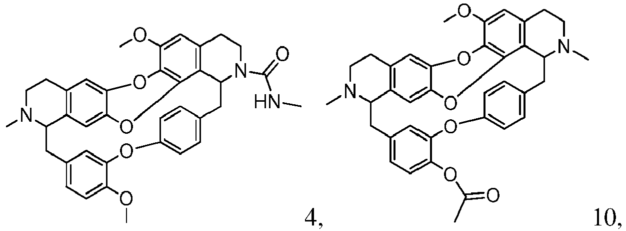

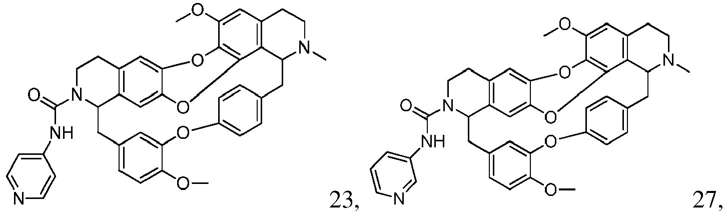

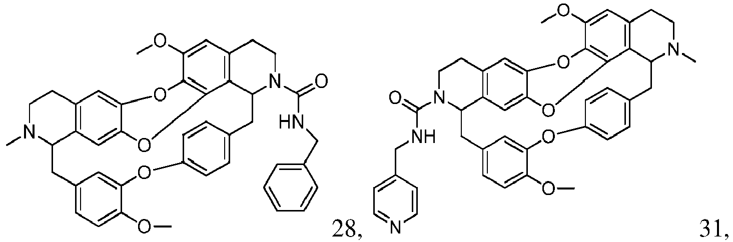

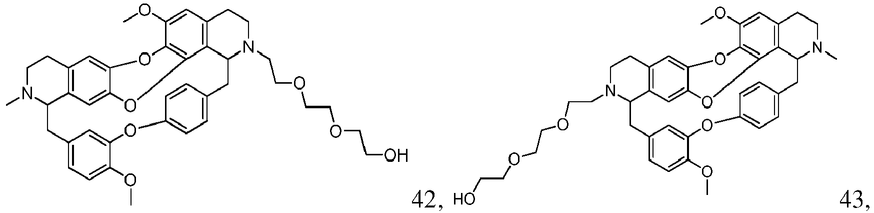

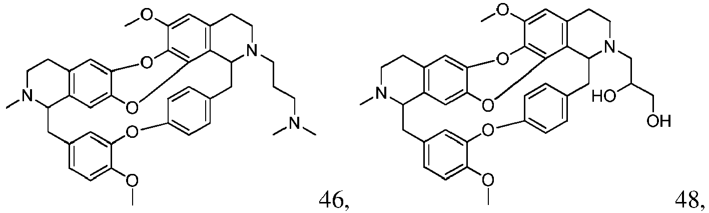

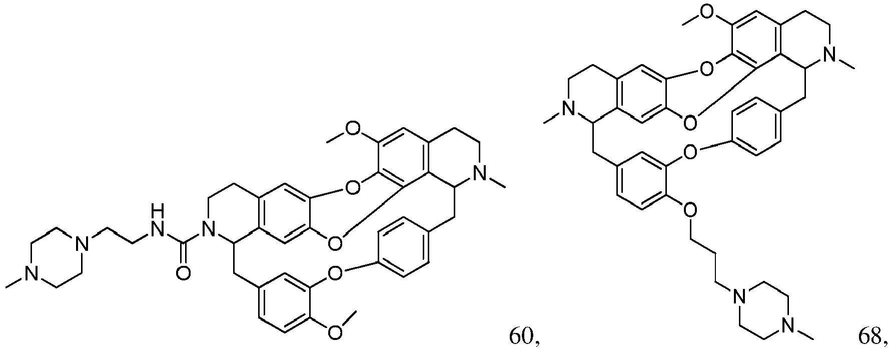

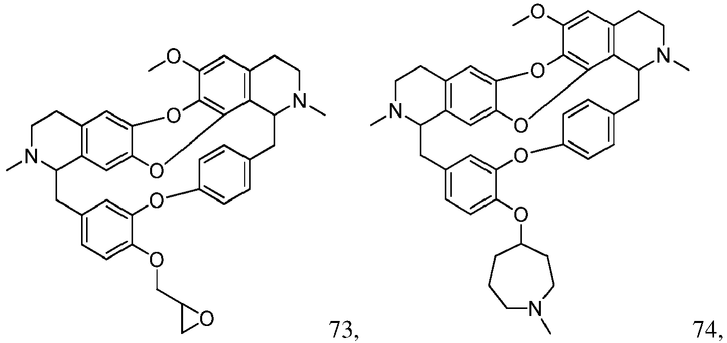

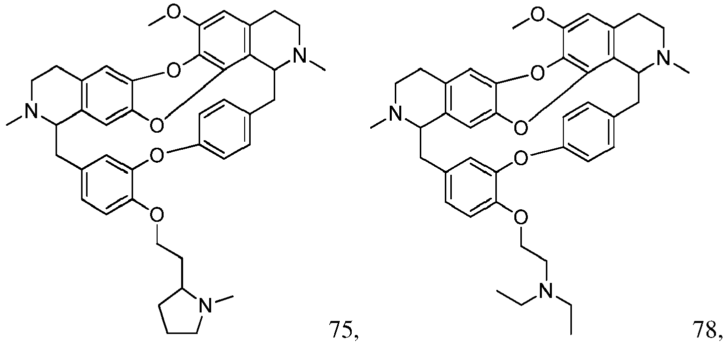

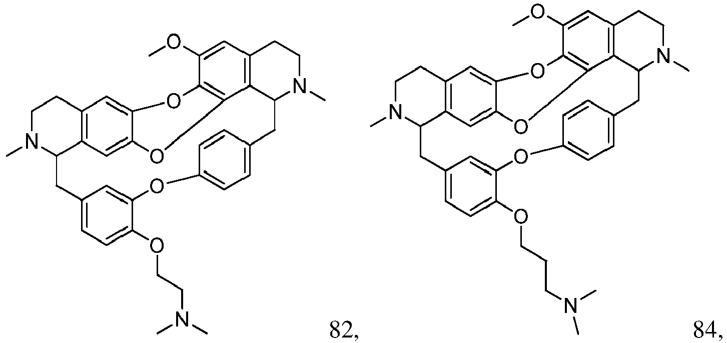

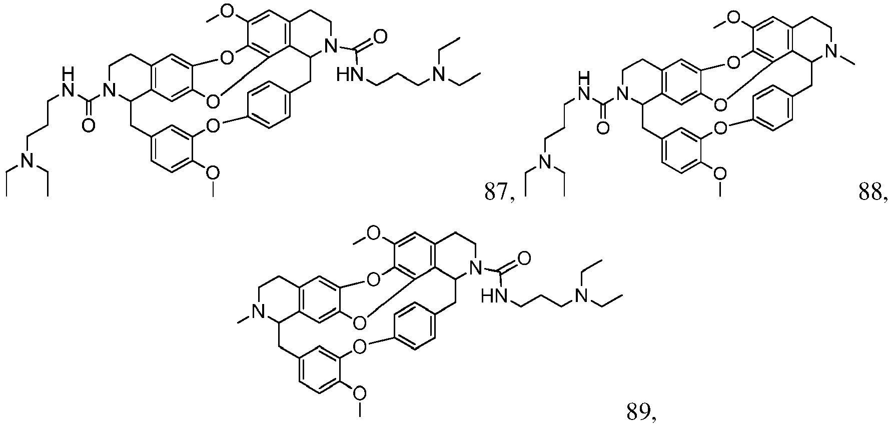

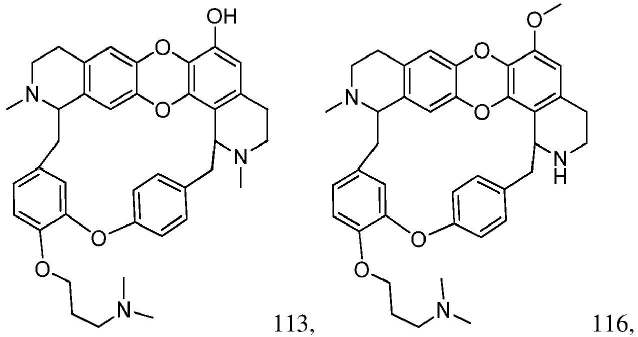

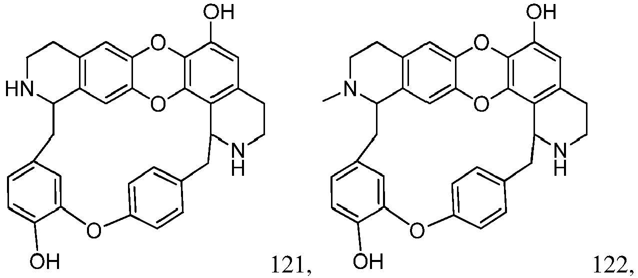

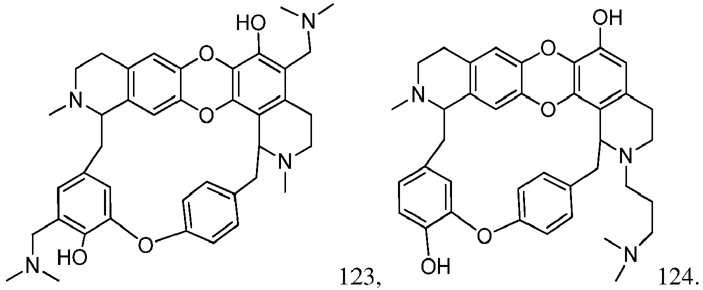

In a particular embodiment, the malaria is caused by a parasite that is a drug-sensitive or drug-resistant parasite, in particular a drug-resistant malaria parasite.

In a particular embodiment, the drug-sensitive or drug-resistant malaria parasite is a chloroquine-resistant; a chloroquine and pyrimethamine-resistant; a chloroquine, pyrimethamine and mefloquine-resistant; a chloroquine, pyrimethamine, mefloquine and artemisinin-resistant; a chloroquine, pyrimethamine, piperaquine and artemisinin-resistant malaria parasite.

In a particular embodiment, malaria is caused by infection with Plasmodium, in particular by *Plasmodium falciparum, P. vivax, P. ovale,* or *P. malariae, P. knowlesi* more particularly by *Plasmodium falciparum.*

In another aspect, the invention concerns a combination of a compound as defined above with at least one other antimalarial agent for the treatment of malaria with simultaneous administration, separate or spread out over time.

In particular, the other antimalarial agent is selected from chloroquine, artemesin, qinghaosu, 8-aminoquinoline, amodiaquine, arteether, artemether, artemisinin, artesunate, artesunic acid, artelinic acid, atovaquone, azithromycine, biguanide, chloroquine phosphate, chlorproguanil, cycloguanil, dapsone, desbutyl halofantrine, desipramine, doxycycline, dihydrofolate reductase inhibitors, dipyridamole, halofantrine, haloperidol, hydroxychloroquine sulfate, imipramine, mefloquine, penfluridol, phospholipid inhibitors, primaquine, proguanil, pyrimethamine, pyronaridine, quinine, quinidine, quinacrine-artemisinin, sulfonamides, sulfones, sulfadoxine, sulfalene, tafenoquine, tetracycline, tetrandine, triazine, salts and mixture thereof.

In another aspect, the present invention concerns a method for the treatment of malaria comprising the administration to a person in need thereof of an effective dose of a compound as defined above.

According to another aspect, the present invention concerns a pharmaceutical composition comprising:
a compound of formula (III) as defined below, or
a stereoisomeric form, a mixture of stereoisomeric forms or a pharmaceutically acceptable salt form thereof,
in admixture with at least one pharmaceutically acceptable excipient.

The compound of formula (III) or the pharmaceutical composition of the present invention may be administered in the form of a conventional pharmaceutical composition by any route including orally, intramuscularly, subcutaneously, topically, intranasally, intraperitoneally, intrathoracially, intravenously, epidurally, intrathecally, intracerebroventricularly and by injection into the joints.

The dosage will depend on the route of administration, the severity of the disease, age and weight of the patient and other factors normally considered by the attending physician, when determining the individual regimen and dosage level at the most appropriate for a particular patient.

For preparing pharmaceutical compositions from the compounds of the present invention, inert, pharmaceutically acceptable carriers can be either solid or liquid. Solid form preparations include powders, tablets, dispersible granules, capsules, cachets, and suppositories.

A solid carrier can be one or more substances, which may also act as diluents, flavouring agents, solubilizers, lubricants, suspending agents, binders, or tablet disintegrating agents; it can also be an encapsulating material.

Tablets, powders, cachets, and capsules can be used as solid dosage forms suitable for oral administration.

Liquid form compositions include solutions, suspensions, and emulsions. For example, sterile water or propylene glycol solutions of the active compounds may be liquid preparations suitable for parenteral administration. Liquid compositions can also be formulated in solution in aqueous polyethylene glycol solution.

Aqueous solutions for oral administration can be prepared by dissolving the active component in water and adding suitable colorants, flavouring agents, stabilizers, and thickening agents as desired. Aqueous solutions for oral use can be made by dispersing the finely divided active component in water together with a viscous material such as natural synthetic gums, resins, methyl cellulose, sodium carboxymethyl cellulose, and other suspending agents known to the pharmaceutical formulation art.

Depending on the mode of administration, the pharmaceutical composition will according to one embodiment of the present invention include 0.05% to 99% weight (percent by weight), according to an alternative embodiment from 0.10 to 50% weight, of the compound of the present invention, all percentages by weight being based on total composition. A therapeutically effective amount for the practice of the present invention may be determined, by the use of known criteria including the age, weight and response of the individual patient, and interpreted within the context of the disease which is being treated or which is being prevented, by one of ordinary skills in the art.

According to another aspect, the present invention concerns a compound of following formula (III): 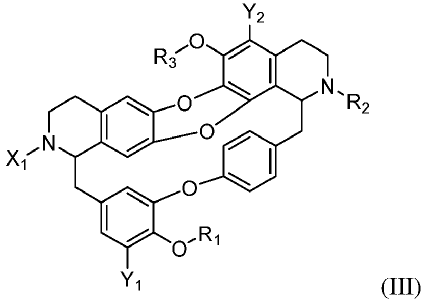 Wherein,
Y₁ and Y₂ are each independently H or (C₁-C₁₀)-alkyl, in which the fragments (C₁-C₁₀)-alkyl are optionally substituted by at least one group selected from NR_{Y1}R_{Y2};
R_{Y1} and R_{Y2} are each independently H or (C₁-C₁₀)-alkyl;
X₁ is H, methyl, ethyl, (C₁-C₁₀)-alkyl-R₄, (C₂-C₁₀)-alkenyl, (C₂-C₁₀)-alkenyl-R₄, CONHR₅, (CH₂-CH₂O)ₘ-H with m=2-4, in which the fragments (C₁-C₁₀)-alkyl are optionally substituted by at least one group selected from halogens;
and N-X₁ optionally represents N⁺-O⁻;
R₂ is chosen from H, methyl, ethyl, (C₁-C₁₀)-alkyl-R₄-, (C₂-C₁₀)-alkenyl, (C₂-C₁₀)-alkenyl-R_{4'}, CONHR_{5'}, (CH₂-CH₂O)ₘ-H with m=2-4, (C₁-C₁₀)-alkyl substituted by at least one group selected from halogen, -OH and -O-(C₁-C₁₀)-alkyl;
R₄ is OH, O-(C₁-C₁₀)-alkyl, O-(C₅-C₁₀)-aryl, NO₂, CN, (3-12)-membered-heterocycle, O-((CH₂)₂O)ₙ-H with n=1-3, CONR_{c}R_{d}, halogen, COOR_{c}, CF₃, or (C₃-C₁₂)-cycloalkyl, in which (C₅-C₁₀)-aryl and (3-12)-membered-heterocycle are optionally substituted by at least one group selected from NR_{c}R_{d}, ORₑ, (C₁-C₁₀)-alkyl, halogen and oxo (=O);
R₅ is H, or a group chosen among (C₁-C₁₀)-alkyl-R₆, (C₃-C₁₂)-cycloalkyl, (C₂-C₁₀)-alkenyl, and a (3-12)-membered-heteroaryl, said group being optionally substituted by at least one group selected from halogen, CN, NR_{c}R_{d}, NO₂, CONR_{c}R_{d}, COOR_{c}, CF₃, ORₑ,
R_{4'} is OH, O-(C₁-C₁₀)-alkyl, O-(C₅-C₁₀)-aryl, NO₂, CN, NR_{c}R_{d}, (3-12)-membered-heterocycle, O-((CH₂)₂O)ₙ-H with n=1-3, CONR_{c}R_{d}, halogen, COOR_{c}, CF₃, or (C₃-C₁₂)-cycloalkyl, in which (C₅-C₁₀)-aryl and (3-12)-membered-heterocycle are optionally substituted by at least one group selected from NR_{c}R_{d}, OR_{c}, (C₁-C₁₀)-alkyl, halogen and oxo (=O);
R_{5'} is H, or a group chosen among (C₁-C₁₀)-alkyl-R_{6'}, (C₃-C₁₂)-cycloalkyl, (C₂-C₁₀)-alkenyl, benzyl, and, only when R₁ and X₁ are not H, Me, said groups being optionally substituted by at least one group selected from halogen, CN, NR_{c}R_{d}, NO₂, CONR_{c}R_{d}, COOR_{c}, CF₃, ORₑ;
R₆ is NR_{c}R_{d}, (3-12)-membered-heteroaryl or (3-12)-membered-heterocycle, in which the fragment (3-12)-membered-heteroaryl or (3-12)-membered-heterocycle is optionally substituted by at least one group selected from NR_{c}R_{d}, ORₑ, (C₁-C₁₀)-alkyl, (C₁-C₁₀)-alkyl-(3-12)-membered-heterocycle, halogen and oxo;
R_{6'} is NR_{c}R_{d} or (3-12)-membered-heterocycle, in which the (3-12)-membered-heterocycle is optionally substituted by at least one group selected from (C₁-C₁₀)-alkyl;
R₁ is H, methyl, -(C₁-C₆) alkyl-NRₐR_{b}, COR₇, (3-12)-membered-azaheterocycle optionally substituted by at least one group selected from (C₁-C₁₀)-alkyl, or (C₁-C₆)-alkyl substituted by at least one group selected from epoxide and (3-12)-membered-azaheterocycle, both optionally substituted by at least one group selected from (C₁-C₁₀)-alkyl,
Rₐ and R_{b} are independently from each other (C₁-C₆) alkyl or H, at least one of Ra and R_{b} being a (C₁-C₆) alkyl;
R_{c}, R_{d} and Rₑ are for each occurence independently from each other (C₁-C₆) alkyl or H; R₃ is H, (C₁-C₆) alkyl, (C₁-C₆) alkyl substituted by NR_{c}R_{d};
R₇ is (C₁-C₁₀)-alkyl, (C₅-C₁₀)-aryl;

With the proviso that:
- R₃ is H, and R₂ is H; or
- At least one of Y₁ and Y₂ is not H; or
- At least one of R₁, R₂, R₃ and X₁ is not H or methyl, with R₁, R₂, R₃ and X₁ being such as:
   X₁ is H, methyl, CONHR₅, (CH₂-CH₂O)ₘ-H with m=2-4, in which the fragments (C₁-C₁₀)-alkyl are optionally substituted by at least one group selected from halogens;
   R₂ is chosen from H, methyl, (C₁-C₁₀)-alkyl-R₄-, CONHR_{5'}, (CH₂-CH₂O)ₘ-H with m=2-4, (C₁-C₁₀)-alkyl substituted by at least one or two groups selected from halogen, -OH and -O-(C₁-C₁₀)-alkyl, in particular -OH;
   R₅ is a group chosen among (C₁-C₁₀)-alkyl-R₆, and a (3-12)-membered-heteroaryl, in particular pyridyl, said group being optionally substituted by at least one group selected from halogen, CN, NR_{c}R_{d}, NO₂, CONR_{c}R_{d}, COOR_{c}, CF₃, ORₑ,
   R_{4'} is NR_{c}R_{d};
   R_{5'} is H, or a group chosen among (C₁-C₁₀)-alkyl-R_{6'} and benzyl, said groups being optionally substituted by at least one group selected from halogen, CN, NR_{c}R_{d}, NO₂, CONR_{c}R_{d}, COOR_{c}, CF₃, ORₑ;
   R₆ is (3-12)-membered-heteroaryl, in particular pyridyl, or (3-12)-membered-heterocycle, in particular piperazinyl, in which the fragment (3-12)-membered-heteroaryl or (3-12)-membered-heterocycle is optionally substituted by at least one group selected from NR_{c}R_{d}, ORₑ, (C₁-C₁₀)-alkyl, (C₁-C₁₀)-alkyl-(3-12)-membered-heterocycle, halogen and oxo;
   R_{6'} is (3-12)-membered-heterocycle, in which the (3-12)-membered-heterocycle is optionally substituted by at least one group selected from (C₁-C₁₀)-alkyl;
   R₁ is H, methyl, -(C₁-C₆)alkyl-NRₐR_{b}, COR₇, (3-12)-membered-azaheterocycle, in particular azepanyl, optionally substituted by at least one group selected from (C₁-C₁₀)-alkyl, or (C₁-C₆)-alkyl substituted by at least one group selected from epoxide and (3-12)-membered-azaheterocycle, in particular pyrrolidinyl or piperazinyl, both epoxide and (3-12)-membered-azaheterocycle being optionally substituted by at least one group selected from (C₁-C₁₀)-alkyl,
   Rₐ and R_{b} are independently from each other (C₁-C₆) alkyl or H, at least one of Ra and R_{b} being a (C₁-C₆) alkyl;
   R_{c}, Rd and Rₑ are for each occurence independently from each other (C₁-C₆) alkyl or H; R₃ is H, (C₁-C₆) alkyl, in particular methyl;
   R₇ is methyl or phenyl, in particular methyl.

All embodiments mentioned above, for example related to formula (I) or (II), apply here as well, alone or in combination.

In a particular embodiment, at least one of R₁, R₂, R₃ and X₁ is not H or methyl, with R₁, R₂, R₃ and X₁ being such as:
X₁ is H, methyl, CONHR₅, in which the fragments (C₁-C₁₀)-alkyl are optionally substituted by at least one group selected from halogens;
R₂ is chosen from H, methyl, (C₁-C₁₀)-alkyl-R₄-, CONHR_{5'}, (C₁-C₁₀)-alkyl substituted by at least one or two groups selected from halogen, -OH and -O-(C₁-C₁₀)-alkyl, in particular -OH;
R₅ is a group chosen among -CH₂-pyridyl, (C₁-C₁₀)-alkyl-piperazinyl, and pyridyl, said group being optionally substituted by at least one group selected from NR_{c}R_{d}, ORₑ, (C₁-C₁₀)-alkyl, (C₁-C₁₀)-alkyl-(3-12)-membered-heterocycle, halogen and oxo;
R_{4'} is N(CH₃)₂;
R_{5'} is H, or a group chosen among (C₁-C₁₀)-alkyl-R_{6'} and benzyl, said groups being optionally substituted by at least one group selected from halogen, CN, NR_{c}R_{d}, NO₂, CONR_{c}R_{d}, COOR_{c}, CF₃, ORₑ;
R_{6'} is (3-12)-membered-heterocycle, in which the (3-12)-membered-heterocycle is optionally substituted by at least one group selected from (C₁-C₁₀)-alkyl;
R₁ is H, methyl, -(C₁-C₆)alkyl-NRₐR_{b}, azepanyl, optionally substituted by at least one group selected from (C₁-C₁₀)-alkyl, or (C₁-C₆)-alkyl substituted by at least one group selected from epoxide and pyrrolidinyl or piperazinyl, both epoxide and (3-12)-membered-azaheterocycle being optionally substituted by at least one group selected from (C₁-C₁₀)-alkyl,
Rₐ and R_{b} are independently from each other (C₁-C₆) alkyl or H, at least one of Rₐ and R_{b} being a (C₁-C₆) alkyl;
R_{c}, Rd and Rₑ are for each occurence independently from each other (C₁-C₆) alkyl or H; R₃ is H, (C₁-C₆) alkyl, in particular methyl.

In a particular embodiment, the compound as defined above is selected from the following compounds:

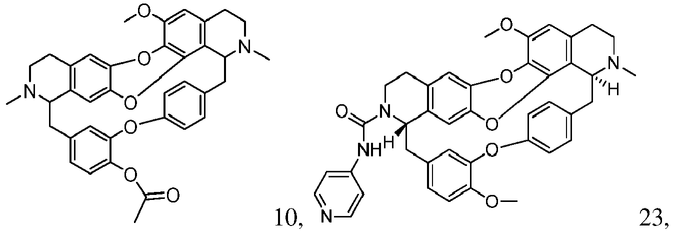

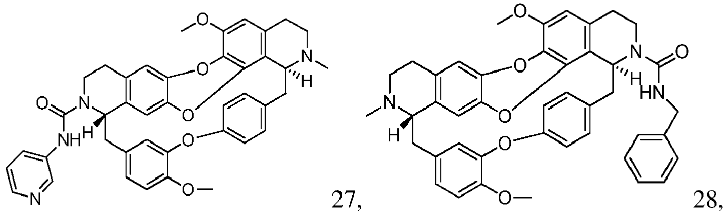

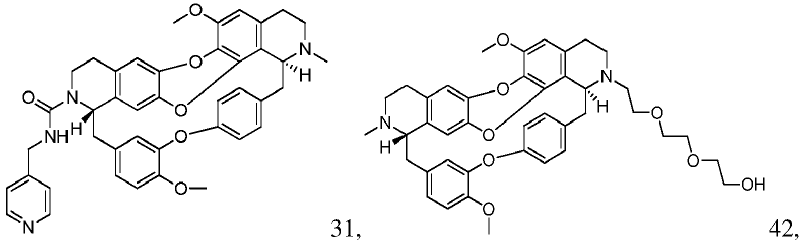

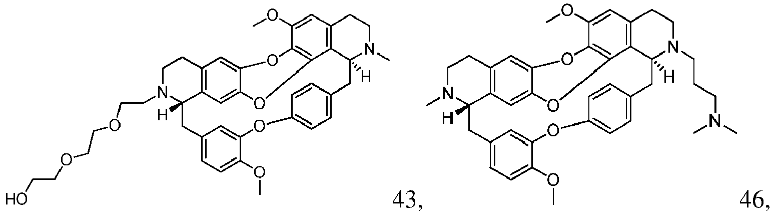

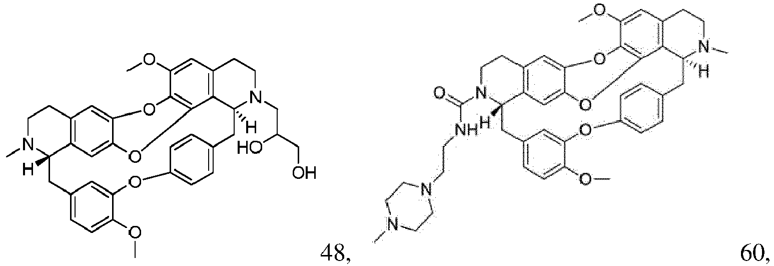

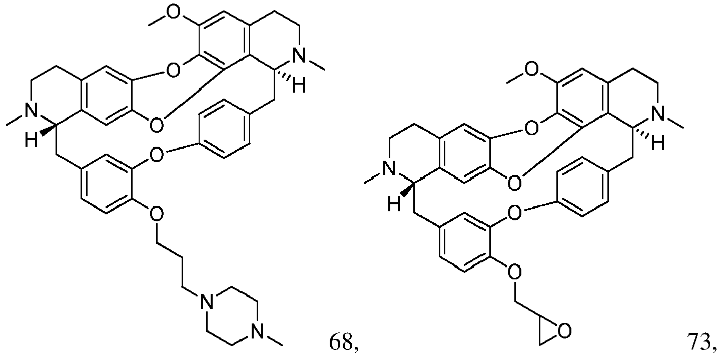

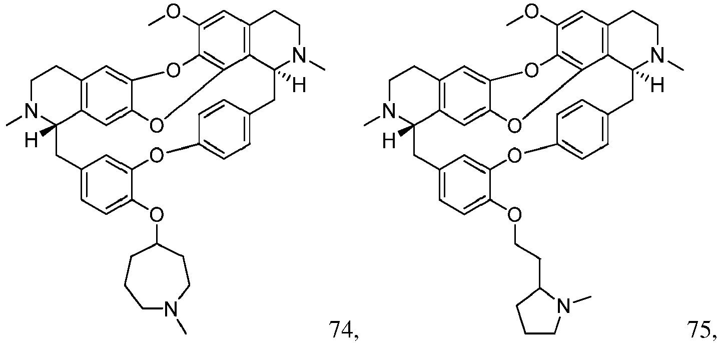

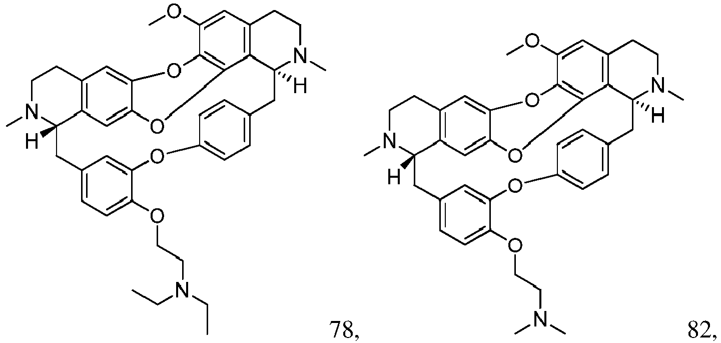

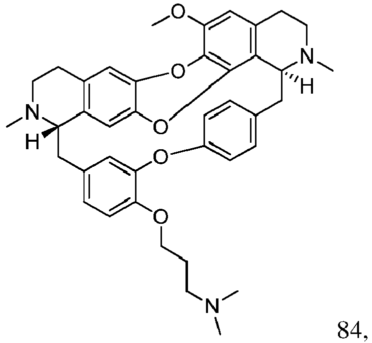

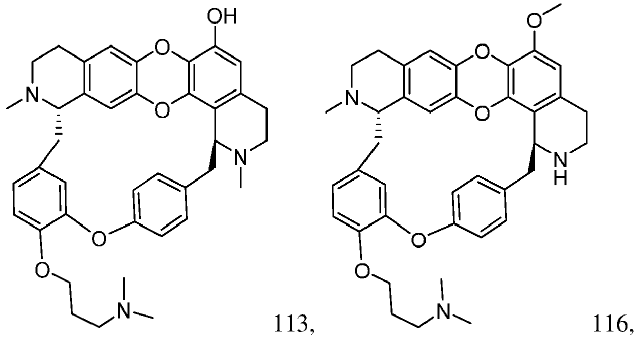

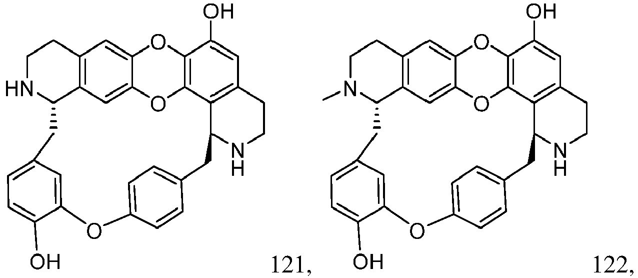

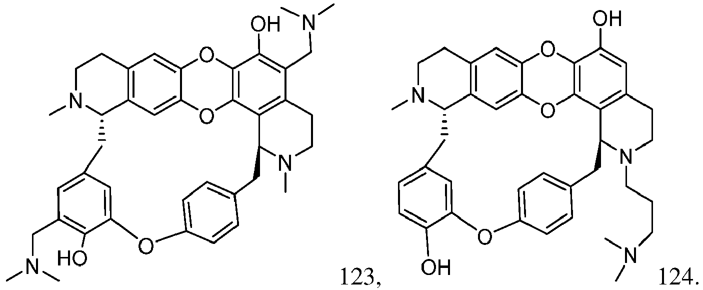

### Definitions

The following terms and expressions contained herein are defined as follows:
As used herein, a range of values in the form "x-y" or "x to y", or "x through y", include integers x, y, and the integers therebetween. For example, the phrases "1-6", or "1 to 6" or "1 through 6" are intended to include the integers 1, 2, 3, 4, 5, and 6. Preferred embodiments include each individual integer in the range, as well as any subcombination of integers. For example, preferred integers for "1-6" can include 1, 2, 3, 4, 5, 6, 1-2, 1-3, 1-4, 1-5, 2-3, 2-4, 2-5, 2-6, etc.

As used herein, the term "patient" or "subject" refers to a warm blooded animal such as a mammal, preferably a human, or a human child, which is afflicted with, or has the potential to be afflicted with one or more diseases and conditions described herein.

By drug-susceptible malaria is meant a case wherein malaria is treated or prevented when the recommended drug dosage is used.

By drug-resistant malaria is meant a case wherein malaria is not treated or prevented when the recommended drug dosage is used.

### Synthesis

The compounds of the present invention may be prepared in a number of methods well known to those skilled in the art, including, but not limited to those described below, or through modifications of these methods by applying standard techniques known to those skilled in the art of organic synthesis. The appropriate modifications and substitutions will be readily apparent and well known or readily obtainable from the scientific literature to those skilled in the art. In particular, such methods can be found in R.C. Larock, Comprehensive Organic Transformations, Wiley-VCH Publishers, 1999.

All processes disclosed in association with the present invention are contemplated to be practiced on any scale, including milligram, gram, multigram, kilogram, multikilogram or commercial industrial scale.

It will be appreciated that the compounds of the present invention may contain one or more asymmetrically substituted carbon atoms, and may be isolated in optically active or racemic forms. Thus, all chiral, diastereomeric, racemic forms, isomeric forms of a structure are intended, unless the specific stereochemistry or isomeric form is specifically indicated. It is well-known in the art how to prepare and isolate such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

Compounds of the present invention may be prepared by a variety of synthetic routes. The reagents and starting materials are commercially available, or readily synthesized by well-known techniques by one of ordinary skill in the arts. All substituents, unless otherwise indicated, are as previously defined.

In the reactions described hereinafter, it may be necessary to protect reactive functional groups, for example hydroxy, amino, imino, thio or carboxy groups, where these are desired in the final product, to avoid their unwanted participation in the reactions. Conventional protecting groups may be used in accordance with standard practice, for examples see T.W. Greene and P. G. M. Wuts in Protective Groups in Organic Chemistry, 3rd ed., John Wiley and Sons, 1999; J. F. W. McOmie in Protective Groups in Organic Chemistry, Plenum Press, 1973.

The reagents and starting materials are commercially available, or readily synthesized by well-known techniques by one of ordinary skill in the arts.

In particular, the compounds defined above are obtained according to the procedures described in patent applications WO2017/055632 and WO2017/055633.

### FIGURES

**Figure 1** illustrates the assay of stage-specificity of compound 84 during the asexual cell cycle, as described in example 3.
**Figure 2** illustrates the ring-stage survival (RSA) assays of compound 84, as described in example 4.
**Figure 3** illustrates the speed of action of compounds 84 and 121 compared to dihydroartemisinin (DHA) and pyrimethamine (PYR), as described in example 5.
**Figure 4** illustrates the *in vivo* activity of compound 84 according to the invention, as described in example 6.

### EXAMPLES

### Synthesis of compounds of the invention

### Synthesis of 84

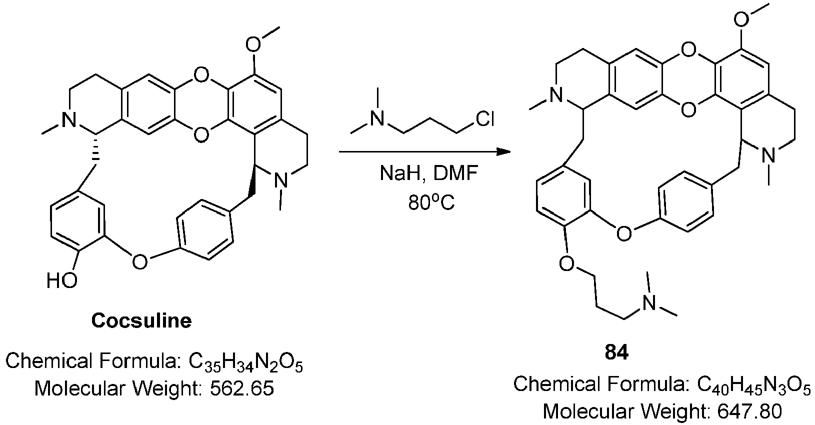

To a suspension of NaH (90%) (11.7 mg, 0.44 mmol, 5.0 equiv) in dry DMF (1.0 mL) was added Cocsuline (50 mg, 0.088, 1.0 equiv) in dry DMF (2.0 mL) at 0 °C and was stirred for 30 min at RT. To the reaction mixture 3-dimethylaminopropylchloride hydrochloride (41.9 mg, 0.26 mmol, 3.0 equiv) and a crystal of KI (1.0 mg) was added and stirred at 80 °C overnight.. The reaction mixture was quenched by drops of a saturated solution of NaHCO₃ and the volatiles were removed under reduced pressure. The residue was partitioned between ethyl acetate and a saturated solution of NaHCO₃. The organic layer was separated and the aqueous layer was extracted 3 time with EtOAc. The combined organic layers were dried over Na₂SO₄ and the solvent was removed under reduced pressure. The residue was purified by silica gel flash chromatography using a linear gradient of DCM/methanol (NH3) (0 to 20%). The desired product **84** was obtained as a yellow powder (41 mg, 72%).
**R_{f}** = 0.51 (DCM/(MeOH/NH₃ 10%) 90/10).
**¹H NMR** (DMSO-*d₆*, 500 MHz): δ (ppm) = 7.62 (d, 1H, *J* = 10 Hz), 7.25-7.19 (m, 2H), 6.89-6.88 (m, 1H), 6.67 (s, 1H), 6.53 (s, 1H), 6.47 (s, 1H), 6.01 (s, 1H), 4.12 (t, 2H, *J* = 5 Hz), 3.96 (s br, 1H), 3.81 (s, 3H), 3.35 (s, br, 2H), 3.28 (d, 1H, *J* = 15 Hz), 3.09 (s, br, 1H), 3.06-3.02 (m, 1H), 2.90-2.79 (m, 4H), 2.64-2.59 (m, 4H), 2.44 (t, 3H, *J* = 5 Hz), 2.53-2.50 (m, 4H), 2.31 (s, 6H), 2.19 (s, 3H), 1.96-1.91 (m, 2H).
**¹³C NMR** (DMSO-*d₆*, 125 MHz): δ (ppm) = 156.4, 153.1, 149.0, 148.7, 142.9, 141.9, 141.9, 141.5, 138.6, 137.9, 134.2, 133.4, 131.6, 131.3, 131.0, 125.4, 124.8, 124.7, 123.3, 119.6, 116.5, 110.1, 70.4, 70.1, 62.7, 58.9, 58.8, 58.0, 52.9, 48.3, 47.8, 45.89, 44.70, 44.19, 30.63, 30.19, 26.64.
**MS** (ESI): [M+H]⁺ C₄₀H₄₆N₃O₅: calcd. 648.3, found 648.3.

Compounds 73, 74, 75, 78 and 82 were obtained similarly, using the corresponding R-Cl starting material instead of 3-dimethylaminopropylchloride hydrochloride.

Compound 10 was obtained by acetylation of cocsuline by methods well known from the skilled in the art.

### Synthesis of Nor-Trilobine

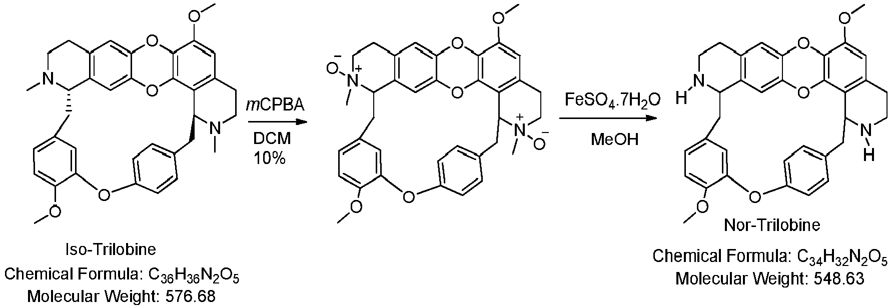

**Step 1:** To a stirred solution containing Iso-trilobine (100 mg, 0.17 mmol, 1.0 equiv) in DCM (10 mL) was added mCPBA 77% (85 mg, 0.38 mmol, 2.2 eq.). The reaction mixture was stirred at RT for 2 h, then diluted with DCM and a saturated solution of NaHCO₃. The organic layer was separated and the aqueous layer was extracted 3 times with EtOAc . The combined organic layers were dried over Na₂SO₄ then concentrated under reduced pressure. The residue was used without further purification.
**Step 2:** To a stirred solution of the crude product of Step 1 in MeOH at 0°C (25.0 mL) was added FeSO₄.7H₂O (144.3 mg, 0.51 mmol, 3.0 equiv). The reaction mixture was stirred at RT overnight, then concentrated under reduced pressure and the residue was dissolved with EDTA (5 mL - 0.1M), adjusted to pH=10 with NH3 solution and extracted 3 times with EtOAc . The combined organic layers were dried over Na₂SO₄ then concentrated under reduced pressure to give a crude mixture of main three products (the starting Iso-trilobine, Trilobine and Nor-trilobine). The crude residue was purified by silica gel chromatography using a linear gradient of DCM/methanol (NH3) (0 to 20%) to give the desired Nor-Trilobine product (30 mg, 32%). The spectral data is in agreement with the reported natural product.
**¹H NMR** (Methanol-*d*₄, 500 MHz): δ (ppm) = 7.58 (dd, 1H, *J* = 8.4, 2.2 Hz), 7.20 (dd, 1H, *J* = 8.3, 2.6 Hz), 7.07 (dd, 1H, *J* = 8.3, 2.2 Hz), 7.04 (d, 1H, *J* = 8.4 Hz), 7.02 (dd, 1H, *J* = 8.3, 2.0 Hz), 6.74 (dd, 1H, *J* = 8.4, 2.5 Hz), 6.62 (s, 1H), 6.61 (d, 1H, *J* = 1.8 Hz), 6.57 (s, 1H), 6.27 (s, 1H), 4.37 (dd, 1H, *J* = 5.8, 1.5 Hz), 3.91 (s, 3H), 3.80 (m, 1H), 3.79 (s, 3H), 3.45-3.34 (m, 3H), 3.19 (m, 1H), 3.12-2.98 (m, 3H), 2.93-2.88 (m, 3H), 2.83 (m, 1H), 2.71 (m, 1H).
   **¹³C NMR** (Methanol-*d*₄, 125 MHz): δ (ppm) = 157.3, 151.3, 149.8, 148.7, 142.0, 141.0, 139.9, 138.4, 133.8, 133.5, 132.7, 130.9, 130.5, 128.6, 124.2, 123.6, 122.3, 119.6, 119.2, 117.2, 114.8, 114.6, 108.9, 59.7, 57.0, 56.9, 54.3, 44.2, 42.6, 40.8, 38.2, 27.6, 26.9.
   **HRMS** (ESI): [M+H]⁺ C₃₄H₃₃N₂O₅: calcd. 549.2384, found 549.2388.

### Synthesis of 121

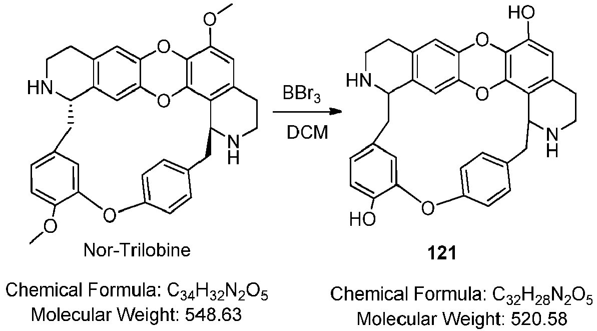

To a stirred solution containing Nor-Trilobine (56 mg, 0.10 mmol, 1.0 eq.) in dry DCM (10.0 mL) was added BBr₃ (1M in DCM) (0.30 mL, 0.30 mmol, 3.0 eq.). The reaction mixture was stirred at RT for 16 h then was diluted with DCM and a saturated solution of NaHCO₃. The organic layer was separated and the aqueous layer extracted 3 times with EtOAc. The combined organic layers were dried over Na₂SO₄ and concentrated under reduced pressure. The residue was purified by silica gel flash chromatography using a linear gradient (DCM/MeOH (NH3) 70/30) to give the desired product **121** (52 mg, 98%).
**R_{f}** = 0.32 (DCM/(MeOH/NH₃ 10%) 80/20).
**¹H NMR** (DMSO-*d₆*, 500 MHz): δ (ppm) 10.02 (s br, 1H), 9.45 (s, br, 2H), 7.35 (d, 1H, *J* = 10 Hz), 7.27-7.25 (m, 1H), 7.09-7.08 (m, 1H), 6.99-6.98 (m, 1H), 6.91-6.88 (m, 2H), 6.73-6.71 (m, 1H), 6.59 (m, 1H), 6.49 (s, 1H), 6.35 (s, 1H), 4.45 (d, 1H, *J* = 5 Hz), 4.01 (d, 1H, *J* = 10 Hz), 3.46-3.37 (m, 4H), 3.17 (d, 1H, *J* = 5 Hz), 3.09-2.79 (m, 8H).
**¹³C NMR** (DMSO-*d₆*, 125 MHz): δ (ppm) 158.9, 150.5, 148.9, 148.2, 143.4, 141.9, 141.3, 137.7, 135.1, 134.2, 133.2, 131.8, 131.1, 129.6, 126.8, 125.1, 123.6, 122.0, 120.3, 118.9, 116.2, 114.6, 59.1, 58.0, 54.5, 51.6, 38.7, 38.4, 27.6, 26.5.
**MS** (ESI): [M+H]⁺ C₃₂H₂₉N₂O₅: calcd. 521.6, found 521.5.

### Synthesis of 46

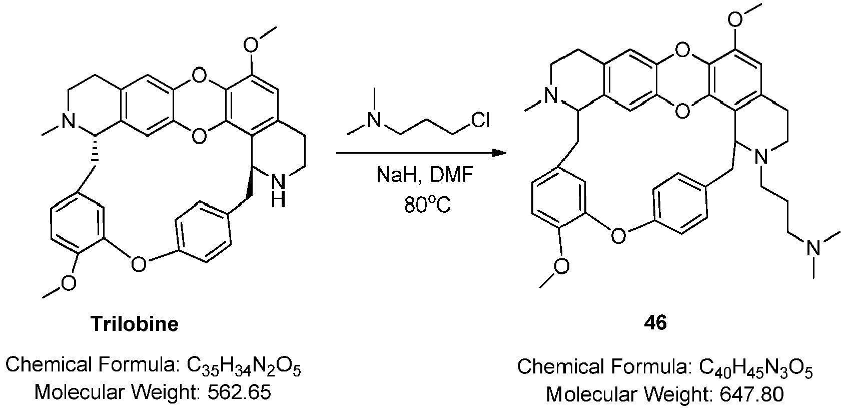

To a suspension of NaH (90%) (50 mg, 0.088 mmol, 5.0 equiv) in dry DMF (1.0 mL) was added Trilobine (50 mg, 0.088, 1.0 equiv) in dry DMF (2.0 mL) at 0 °C. The reaction mixture was stirred for 30 min and 3-dimethylaminopropylchloride hydrochloride (41.9 mg, 0.26 mmol, 3.0 equiv) and a crystal of KI (1.0 mg) were added. The reaction mixture was stirred at 80°C overnight. The reaction mixture was quenched by drops of a saturated solution of NaHCO₃ and the volatiles were removed under reduced pressure. The residue was partitioned between EtOAc and a saturated solution of NaHCO₃. The organic layer was separated and the aqueous layer extracted 3 times with EtOAc. The combined organic layers were dried over Na₂SO₄ and concentrated under reduced pressure. The residue was purified by silica gel flash chromatography using a linear gradient of DCM/methanol (NH3) (0 to 20%). The desired product was obtained **46** as a yellow powder (49 mg, 85%).
**R_{f}** = 0.74 (DCM/(MeOH/NH₃ 10%) 90/10).
**¹H NMR** (DMSO-*d₆*, 500 MHz): δ (ppm) 7.59 (d, 1H, *J* = 10 Hz), 7.21 (d, 1H, *J* = 10 Hz), 7.18-7.16 (dd, 1H, *J* = 5, 10 Hz), 7.01-6.99 (dd, 1H, *J* = 5, 10 Hz), 6.96 (d, 1H, *J* =10 Hz), 6.83 (d, 1H, *J* =10 Hz), 6.61 (s, 1H), 6.48 (s, 1H), 6.44 (s, 1H), 5.93 (s, 1H), 3.90 (s, br, 1H), 3.84 (s, 3H), 3.79 (s, 3H), 3.49-3.31 (m, 3H), 3.42-3.21 (d, 1H, *J* = 15), 3.00-2.96 (m, 1H), 2.87-2.77 (m, 4H), 2.60-2.55 (m, 4H), 2.47 (s, 3H), 2.33-2.24 (m, 3H), 2.18 (s, 6H), 1.51-1.44 (m, 2H).
**¹³C NMR** (DMSO-*d₆*, 125 MHz): δ (ppm) 153.1, 149.6, 146.5, 145.5, 139.9, 138.9, 138.6, 135.8, 134.9, 131.2, 130.3, 128.9, 128.5, 128.1, 122.2, 121.5, 121.4, 120.0, 116.0, 115.3, 113.5, 112.5, 64.9, 59.6, 56.6, 55.8, 55.7, 50.1, 44.9, 44.7, 44.5, 41.5, 41.1, 40.9, 26.7, 24.1, 23.5.
MS (ESI): [M+H]⁺ C₄₀H₄₆N₃O₅: calcd. 648.3, found 648.3.

### Synthesis of 110

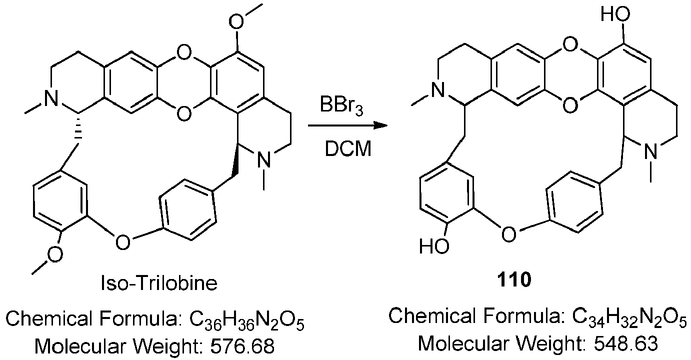

To a stirred solution containing Iso-Trilobine (50 mg, 0.086 mmol, 1.0 eq.) in dry DCM (10.0 mL) was added BBr₃ (1M in DCM) (0.26 mL, 0.26 mmol, 3.0 eq.). The reaction mixture was stirred at RT for 16 h, then diluted with DCM and saturated solution of NaHCO₃. The organic layer was separated and the aqueous layer extracted 3 times with EtOAc. The combined organic layers were dried over Na₂SO₄ and concentrated under reduced pressure. The residue was purified by silica gel flash chromatography using a linear gradient (DCM/MeOH (NH3) 80/20) to give the desired product **110** (42 mg, 89%).
**R_{f}** = 0.35 (DCM/(MeOH/NH₃ 10%) 80/20).
**¹H NMR** (DMSO-*d₆*, 500 MHz): δ (ppm) 9.48 (s br, 1H), 9.32 (s br, 1H), 7.57 (d, 1H, *J* = 10 Hz), 7.20-7.15 (m, 2H), 7.02 (d, 1H, *J* = 5 Hz), 6.81-6.79 (m, 1H), 6.75-6.73 (m, 1H), 6.60 (s, 1H), 6.41 (s, 1H), 6.30 (s, 1H), 6.00 (s, 1H), 4.08 (s br, 1H), 3.90 (s br, 1H), 3.26-3.23 (d, 1H, *J* = 15 Hz), 3.17 (s, 2H), 3.09 (s br, 1H), 3.02-2.98 (m, 1H), 2.85-2.74 (m, 3H), 2.67-2.54 (m, 3H), 2.49 (s, 3H), 2.42-2.36 (m, 1H), 2.28 (s, 3H).
**¹³C NMR** (DMSO-*d₆*, 125 MHz): δ (ppm) 156.8, 151.7, 147.4, 146.5, 142.7, 142.1, 142.0, 141.7, 136.0, 134.1, 133.2, 131.3, 131.0, 130.7, 125.4, 125.0, 124.6, 119.8, 119.5, 118.3, 116.5, 113.7, 70.4, 62.7, 58.0, 53.0, 51.7, 47.7, 44.6, 44.1, 30.4, 26.3. **MS** (ESI): [M+H]⁺ C₃₄H₃₃N₂O₅: calcd. 549.6, found 549.6.

### Synthesis of 113

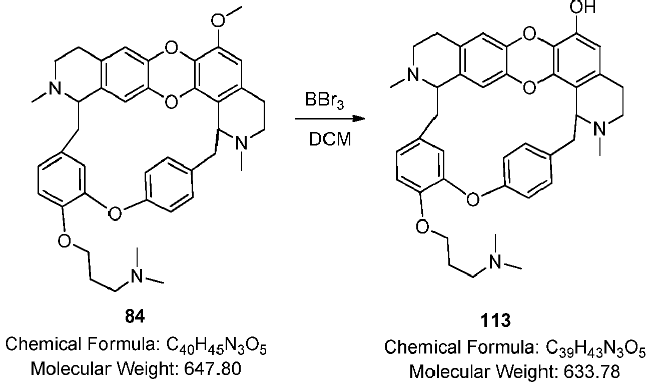

To a stirred solution of compound **84** (10 mg, 0.015mmol, 1.0 eq.) in dry DCM (3.0 mL) was added BBr₃ (1M in DCM) (0.045 mL, 0.045 mmol, 3.0 eq.). The reaction mixture was stirred at RT for 16 h and was then diluted with DCM and a saturated solution of NaHCO₃. The organic layer was separated and the aqueous layer extracted 3 times with EtOAc. The combined organic layers were dried over Na₂SO₄ and concentrated under reduced pressure. The residue was purified by silica gel flash chromatography using a linear gradient (DCM/MeOH (NH3) 80/20) to give the desired product **113** (2 mg, 25%).
**R_{f}** = 0.35 (DCM/(MeOH/NH₃ 10%) 80/20).
**¹H NMR** (DMSO-*d₆*, 500 MHz): δ (ppm) = 9.48 (s br, 1H), 7.58 (d, 1H, *J* = 10 Hz), 7.21 (d, 1H, *J* = 10 Hz), 7.18-7.16 (dd, 1H, *J* = 5, 10 Hz), 7.04-7.02 (dd, 1H, *J* = 5, 10 Hz), 6.98 (d, 1H, *J* = 10 Hz), 6.68 (d, 1H, *J* = 10 Hz), 6.60 (s, 1H), 6.44 (s, 1H), 6.29 (s, 1H), 5.98 (s, 1H), 4.09 (t, 2H, *J* = 5 Hz), 3.90 (s br, 1H), 3.06 (s br, 1H), 3.01-2.98 (m, 1H), 2.88-2.85 (m, 1H), 2.78-2.72 (m, 3H), 2.63-2.56 (m, 4H), 2.48 (s, 3H), 2.44-2.36 (m, 5H), 2.28 (s, 3H), 2.17 (s, 6H), 1.94-1.88 (m, 2H).
**¹³C NMR** (DMSO-*d₆*, 125 MHz): δ (ppm) 156.3, 153.1, 149.0, 146.4, 144.7, 143.0, 142.1, 142.0, 141.8, 138.4, 137.9, 134.2, 133.2, 131.3, 131.0, 130.8, 124.8, 124.7, 121.7, 119.5, 118.3, 113.7, 70.4, 70.0, 62.7, 58.8, 52.9, 52.1, 48.3, 47.8, 46.4, 45.8, 44.7, 44.2, 30.1, 26.4.
**MS** (ESI): [M+H]⁺ C₃₉H₄₄N₃O₅: calcd. 634.7, found 634.7.

### Synthesis of 122

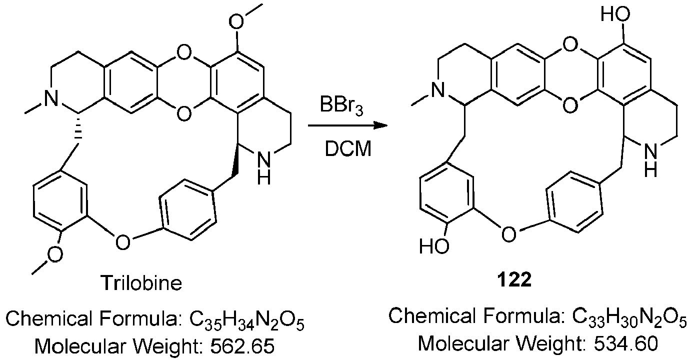

To a stirred solution of Trilobine (50 mg, 0.088 mmol, 1.0 eq.) in dry DCM (10.0 mL) was added BBr₃ (1M in DCM) (0.26 mL, 0.26 mmol, 3.0 eq.). The reaction mixture was stirred at RT for 16 h and then diluted with DCM and saturated solution of NaHCO₃. The organic layer was separated and the aqueous layer extracted 3 times with EtOAc. The combined organic layers were dried over Na₂SO₄ and concentrated under reduced pressure. The residue was purified by silica gel flash chromatography using a linear gradient (DCM/MeOH (NH3) 80/20) to give the desired product **122** (35 mg, 74%).
**R_{f}** = 0.26 (DCM/(MeOH/NH₃ 10%) 80/20).
**¹H NMR** (DMSO-*d₆*, 500 MHz): δ (ppm) = 9.63 (s br, 1H), 9.32 (s br, 1H), 7.68 (d, 1H, *J* = 10 Hz), 7.20-7.19 (m, 2H), 7.02-7.00 (dd, 1H, *J* = 5, 10 Hz), 6.80-6.79 (m, 1H), 6.74-6.72 (m, 1H), 6.58 (s, 1H), 6.39 (s, 1H), 6.32 (s, 1H), 6.02 (s, 1H), 4.34 (s br, 1H), 3.29-3.26 (m, 4H), 3.15 (m, 2H), 3.09 (s br, 2H), 2.45-2.24 (m, 1H), 2.82-2.68 (m, 4H), 2.62-2.53 (m, 3H), 2.28 (s, 3H).
**¹³C NMR** (DMSO-*d₆*, 125 MHz): δ (ppm) = 157.1, 151.6, 147.5, 147.1, 142.0, 141.8, 141.5, 137.5, 136.1, 134.1, 133.3, 131.7, 131.2, 126.0, 125.1, 125.1, 124.5, 119.9, 119.6, 118.3, 116.5, 113.9, 70.3, 55.9, 52.5, 51.7, 45.7, 43.9, 40.5, 33.8, 30.4.
MS (ESI): [M+H]⁺ C₃₃H₃₁N₂O₅: calcd. 535.6, found 535.6.

### Synthesis of 116

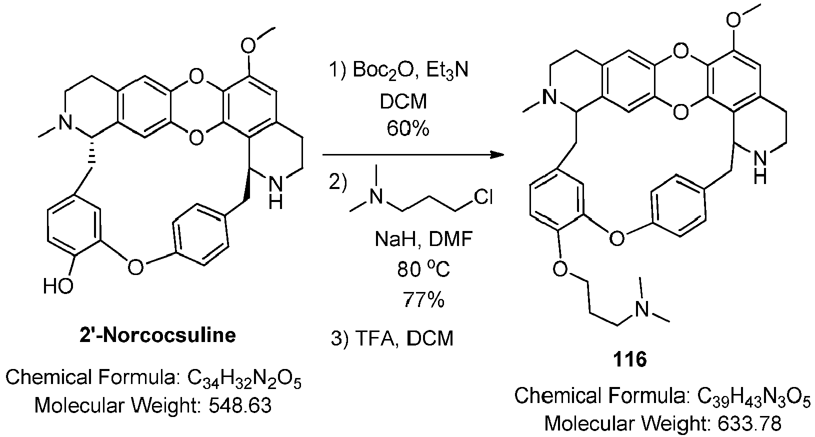

**Step 1:** To a stirred solution containing 2'-NorCocsuline (50 mg, 0.091 mmol, 1.0 equiv) in DCM (5.0 mL), Boc₂O (21.8 mg, 0.10 mmol, 1.1 equiv) and Et3N (15 µL, 0.140 mmol, 1.1 equiv) were added and the reaction mixture was stirred at RT. for 24h. The solvent was evaporated and the residue was purified by silica gel flash chromatography using a linear gradient (DCM/MeOH 95/5) to give the desired protected product (35 mg, 60%).
**Step 2:** To a suspension of NaH (90%) (7.1 mg, 0.26 mmol, 5.0 equiv) in dry DMF (1.0 mL) was added the product of step **1** (35 mg, 0.053, 1.0 equiv) in dry DMF (2.0 mL) at 0 °C. The reaction mixture was stirred at RT for 30 min and then 3-dimethylaminopropylchloride hydrochloride (25.13 mg, 0.26 mmol, 3.0 equiv) and a crystal of KI (1.0 mg) were added. The reaction mixture was stirred at 80 °C overnight and then quenched by drops of a saturated solution of NaHCO₃. Volatiles were removed under reduced pressure and the residue was partitioned between ethyl acetate and saturated solution of NaHCO₃. The organic layer was separated and the aqueous layer extracted 3 times with EtOAc. The combined organic layers were dried over Na₂SO₄ and concentrated under reduced pressure. The residue was purified by silica gel flash chromatography using a linear gradient of DCM/methanol (NH3) (0 to 20%). The desired product was obtained as a yellow powder (30 mg, 77%).
**Step 3:** To a solution of the product of step **2** (10 mg, 0.013 mmol) in DCM (1.0 mL) was added TFA (0.5 mL). The reaction mixture was stirred overnight at RT. The volatiles were evaporated and the residue was purified by silica gel flash chromatography using a linear gradient (DCM/MeOH(NH3) 8/2) to give the desired product as white solid **116** (8 mg, 97%).
   **R_{f}** = 0.19 (DCM/(MeOH/NH₃ 10%) 80/20).
   **¹H NMR** (DMSO-*d₆*, 500 MHz): δ (ppm) = 7.64 (d, 1H, *J* = 5), 7.19-7.18 (m, 2H), 7.01-6.95 (m, 2H), 6.84-6.82 (m, 1H), 6.61 (s, 1H), 6.45-6.42 (m, 2H), 6.01 (s, 1H), 4.16 (s br, 1H), 4.07 (t, 2H, *J* = 5 Hz), 3.75 (s, 3H), 3.18-3.16 (d, 1H, *J* = 15), 3.09 (s br, 1H), 3.00-2.95 (m, 2H), 2.88-2.85 (m, 2H), 2.76-2.96 (m, 3H), 2.61-2.51 (m, 4H), 2.39 (m, 3H), 2.30 (s, 3H), 2.14 (s, 6H), 1.90-1.87 (m, 2H).
   **¹³C NMR** (DMSO-*d₆*, 125 MHz): δ (ppm) 153.3, 149.9, 146.0, 145.4, 140.3, 138.8, 138.0, 135.8, 134.9, 131.1, 130.0, 129.1, 128.4, 128.1, 121.9, 121.8, 121.3, 116.5, 115.2, 114.3, 113.5, 107.2, 67.1, 67.0, 55.8, 55.7, 54.9, 49.0, 45.2, 44.6, 42.8, 41.1, 38.1, 27.7, 27.4, 27.1.
   **MS** (ESI): [M+H]⁺ C₃₉H₄₄N₃O₅: calcd. 634.78, found 634.7.

### Synthesis of 124

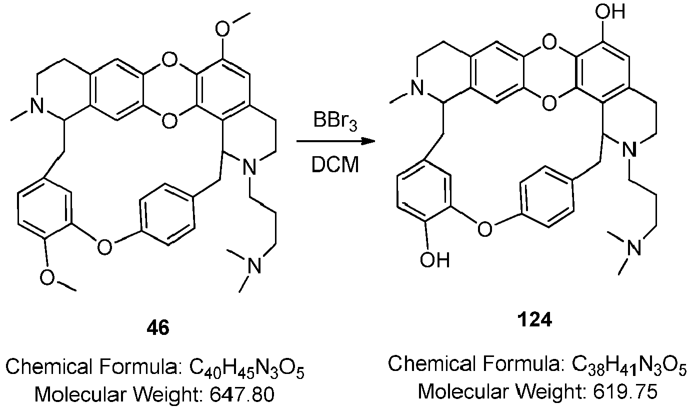

To a stirred solution of **46** (5 mg, 0.0036 mmol, 1.0 eq.) in dry DCM (2.0 mL) was added BBr₃ (1M in DCM) (0.010 mL, 0.010 mmol, 3.0 eq.). The reaction mixture was stirred at room temperature for 16 h and was then diluted with DCM and a saturated solution of NaHCO₃. The organic layer was separated and the aqueous layer extracted with EtOAc 3 times. The combined organic layers were dried over Na₂SO₄ and concentrated under reduced pressure. The residue was purified by silica gel flash chromatography using a linear gradient (DCM/MeOH (NH3) 80/20) to give the desired product **124** (2 mg, 86%).
**R_{f}** = 0.21 (DCM/(MeOH/NH₃ 10%) 80/20).
**¹H NMR** (DMSO-*d₆*, 500 MHz): δ (ppm) 9.50 (s br, 1H), 9.32 (s br, 1H), 7.60 (m, 1H), 7.23-7.21 (m, 2H), 7.02-7.01 (m, 2H), 6.81-6.79 (m, 1H), 6.60 (s, 1H), 6.73 (s, 1H), 6.30 (s, 1H), 5.97 (s, 1H), 4.09 (s br, 2H), 3.89 (s br, 1H), 3.17 (s, 2H), 2.85-.273 (m, 6H), 2.63-2.51 (m, 7H), 2.48 (s, 3H), 2.40 (s, 6H), 2.19-2.16 (m, 2H)
**¹³C NMR** (DMSO-*d₆*, 125 MHz): δ (ppm) 153.6, 148.6, 144.3, 143.4, 139.1, 138.8, 138.8, 135.5, 134.6, 133.3, 131.6, 131.1, 130.2, 130.1, 128.1, 127.9, 123.3, 121.8, 120.1, 116.6, 116.4, 115.2, 69.7, 65.0, 59.7, 56.2, 56.0, 49.7, 48.5, 44.9, 43.6, 41.5, 31.2, 29.0, 28.6.
**MS** (ESI): [M+H]⁺ C₃₈H₄₂N₃O₅: calcd. 620.7, found 620.7.

### Synthesis of 123

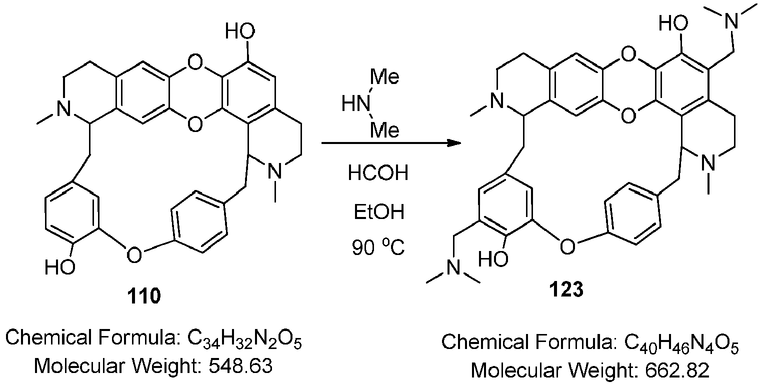

To a stirred solution containing HH_1225 (10 mg, 0.018 mmol, 1.0 equiv) in EtOH (2.0 mL) was added dimethyl amine hydrochloride (7.4 mL, 0.091 mmol, 5.0 equiv) and para formaldhyde (5.40 mg, 0.18 mmol, 10 equiv). The reaction mixture was stirred at 90 °C for 16 h. Solvent was removed under vaccum and the residue was diluted with EtOAc and a solution of saturated NaHCO₃. The organic layer was separated and the aqueous layer extracted 3 times with EtOAc. The combined organic layers were dried over Na₂SO₄ and concentrated under reduced pressure. The residue was purified by silica gel flash chromatography using a linear gradient (DCM/MeOH (NH3) 90/10) to give the desired product **123** (5.0 mg, 60%).
**MS** (ESI): [M+H]⁺ C₄₀H₄₇N₄O₅: calcd. 663.8, found 663.8.

### Synthesis of 87

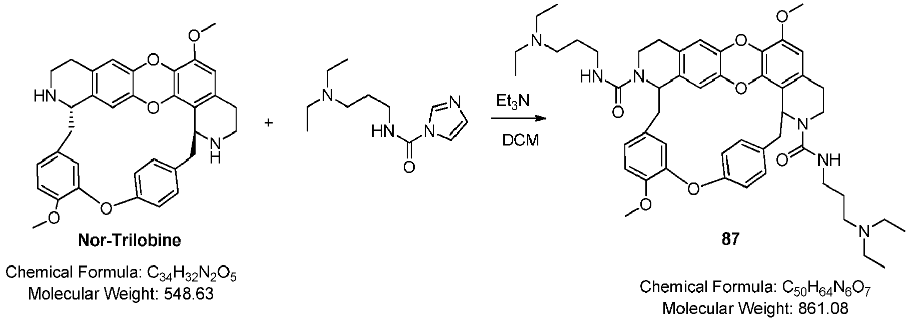

To a solution of nortrilobine (6.3 mg, 11.49 (µmol) in 500 µL of DCM were added *N*-(3-(diethylamino)propyl)-1*H*-imidazole-1-carboxamide (10.32 mg, 46.00 (µmol) and triethylamine (6 µL, 44.44µmol). The mixture was stirred at RT for 24 hours., then concentrated under reduced pressure and the residue was purified by HPLC semi-preparative X-Terra RP-18, eluting with a linear gradient H₂O/MeCN with 0.02% triethylamine (80:20 to 0:100), to give **87** (3.1 mg, 3.60 µmol, 31%).
**1H NMR (500MHz, CDCl₃)** δ 8.16 (1H, br d, *J* = 8.5 Hz,), 7.30 (1H, dd, *J* = 8.4 Hz, *J* = 2.7 Hz), 7.16 (1H, br dd, *J* = 8.2 Hz, *J* = 1.9 Hz), 7.05 (1H, dd, *J* = 8.2 Hz, *J* = 2.5 Hz), 6.90 (1H, d, *J* = 8.3 Hz), 6.79 (1H, dd, *J* = 8.3 Hz, 25 *J* = 1.8 Hz), 6.62 (1H, d, *J* = 2.0 Hz), 6.61 (1H, s), 6.32 (1H, s), 6.11 (1H, s), 5.80 (1H, br), 4.65 (1H, br), 4.26 (1H, br d, *J* = 12.0Hz), 4.01 (1H, m), 3.98 (3H, s), 3.85 (3H, s), 3.44 (4H, m), 3.33 (1H, br d, *J* = 15.5 Hz), 3.19 (1H, m), 3.06-2.92 (5H, m), 2.89-2.76 (8H, m), 2.73 (1H, m), 2.69-2.61 (5H, m), 2.51 (1H, br d, *J* = 16.3 Hz), 1.92 (2H, m), 1.73 (2H, m), 1.16 (12H, m).
**¹³C NMR (125MHz, CDCl₃)** δ 157.3, 157.2, 154.2, 150.8, 147.7, 146.4, 139.9, 139.3, 139.2, 134.6, 131.1, 130.2, 129.8, 128.5, 123.2, 121.8, 121.5, 121.2, 116.8, 116.1, 114.2, 112.3, 106.7, 58.4 , 56.6, 56.3, 52.2, 50.0, 46.5, 46.0, 45.4, 39.2, 38.1, 28.2, 27.4, 25.5, 24.8, 15 9.8, 9.4.
**HRMS-ESI** (m/z) calculated for C₅₀H₆₅N₆O₇ [M+H]⁺: 862.4909; Found: 862.4892.

### Synthesis of 88

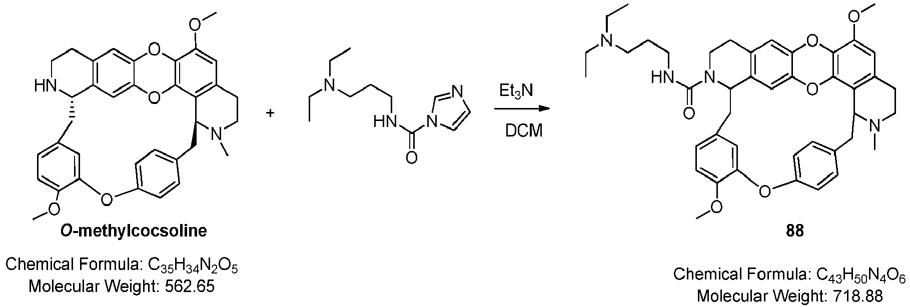

To a solution of *O*-methylcocsoline (10.5 mg, 18.68 (µmol) in 500 µL of THF were added *N*-(3-(diethylamino)propyl)-1*H*-imidazole-1-carboxamide (2.5 mg, 36.55 (µmol) and triethylamine (5 µL, 37.03 µmol). The mixture was stirred at RT for 24 hours. The reaction mixture was concentrated under reduced pressure and the residue was purified by HPLC semi-preparative X-Terra RP-18, eluting with a linear gradient H₂O/MeCN with 0.02% triethylamine (85:15 to 70:30), to give **88** (12 mg, 16.71 µmol, 89%).
**1H NMR (500MHz, CDCl₃)** δ 7.66 (1H, br d, *J* = 7.8 Hz), 7.28 (1H, br), 7.17 (1H, br d, *J* = 7.9 Hz), 7.11 (1H, br d, *J* = 7.9 Hz), 6.91 (1H, d, J = 8.1 Hz), 6.78 (1H, br d, *J* = 7.9 Hz), 6.67 (1H, br), 6.63 (1H, s), 6.33 (1H, s), 6.12 (1H, s), 4.77 (1H, br), 4.26 (1H, br d, *J* = 11.6 Hz), 4.09 (1H, br), 3.98 (3H, s), 3.87 (3H, s), 3.41(1H, br d, *J* = 15.3 Hz), 3.29-3.18 (3H, m,), 3.08-2.94 (8H, m), 2.88 (2H, m,), 2.82-2.73 (3H, m, H-4), 2.63 (3H, s,), 2.54 (2H, m), 1.87 (2H, m,), 1.24 (6H, t, *J* = 7.1 Hz).
**¹³C NMR (125MHz, CDCl₃)** δ 157.2, 154.4, 150.5, 147.8, 146.5, 140.0, 139.8, 139.7, 134.6, 131.5, 130.2, 129.8, 128.4, 127.3, 122.8, 122.0, 121.8, 119.5, 116.9, 116.2, 114.1, 112.5, 106.6, 61.0, 58.0, 56.5, 56.4, 48.9, 46.5, 45.6, 45.2, 42.1, 41.5, 38.2, 38.1, 28.2, 24.7, 23.3, 8.5.
**HRMS-ESI** (m/z) calculated for C₄₃H₅₁N₄O₆ [M+H]⁺: 719.3803; Found: 719.3794.

### Synthesis of 89

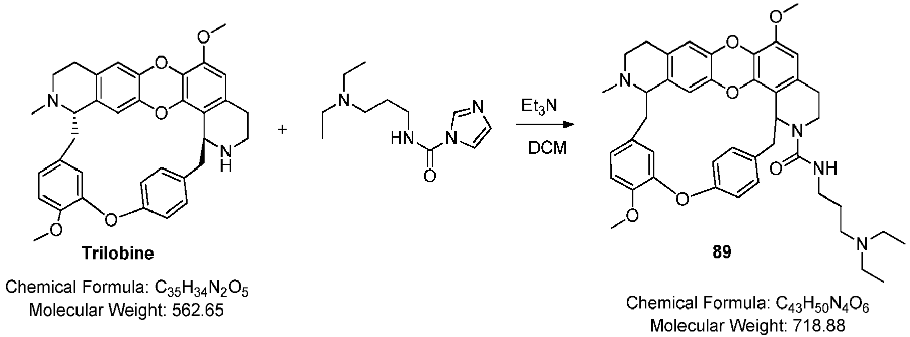

To a solution of trilobine (10.5 mg, 18.68 (µmol) in 500 µL of DCM were added *N*-(3-(diethylamino)propyl)-1*H*-imidazole-1-carboxamide (8.4 mg, 37.44 (µmol) and triethylamine (5 µL, 37.03µmol). The mixture was stirred at RT for 24 hours, then concentrated under reduced pressure and the residue was purified by HPLC semi-preparative X-Terra RP-18, eluting with a linear gradient H₂O/MeCN with 0.1% formic acid (85:15 to 70:30), to give **89** (8 mg, 11.14 µmol, 59%).
**1H NMR (500MHz, CDCl₃)** δ 7.96 (1H, br d, *J* = 7.4 Hz), 7.19 (1H, br d, *J* = 7.4 Hz), 6.97 (2H, br m), 6.88 (2H, br m), 6.67 (1H, s), 6.60 (1H, br), 6.43 (1H, s), 6.35 (1H, s), 5.67 (1H, br d, *J* = 5.8 Hz), 3.94 (3H, s), 3.88 (3H, s), 3.69-3.59 (3H, m), 3.49-3.40 (2H, m3.28 (2H, m), 3.11 (1H, br d, *J* = 14.4 Hz), 3.04-2.97 (6H, m), 2.94-2.83 (4H, m), 2.74- 2.66 (3H, m), 2.35 (3H, s), 2.01 (2H, m), 1.25 (6H, t, *J* = 7.0 Hz).
**¹³C NMR (125MHz, CDCl₃)** δ 157.8, 154.4, 149.8, 147.7, 146.5, 140.6, 139.7, 138.7, 137.6, 132.7, 131.3, 131.0, 130.8, 130.6, 129.9, 129.7, 123.1, 122.8, 121.3, 120.3, 117.4, 115.8, 114.8, 112.5, 106.8, 64.8, 56.6 , 56.3, 51.4, 50.9, 49.1, 49.2, 45.6, 40.9, 39.5, 38.1, 37.3, 28.3, 26.3, 24.6, 8.6.
**HRMS-ESI** (m/z) calculated for C₄₃H₅₁N₄O₆ [M+H]⁺: 719.3803; Found: 719.3794.

### Synthesis of 68

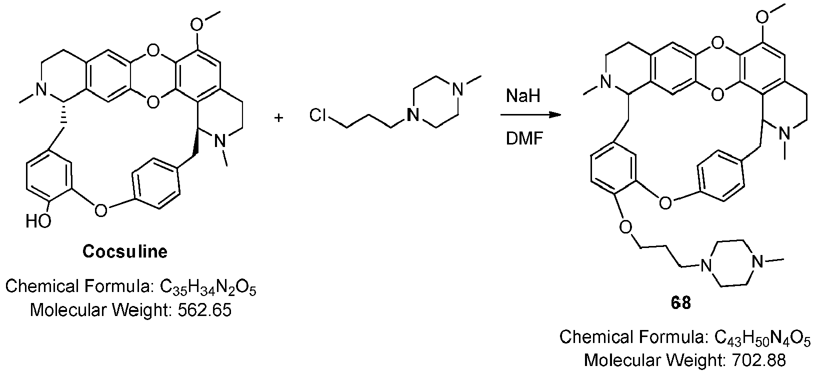

To a solution of NaH (1.28 mg, 0.053 mmol, 3.0 equiv) in DMF (200 µL) were added Cocsuline (10.0 mg, 0.018 mmol, 1.0 equiv) and 1-(3-chloropropyl)-4-methylpiperazine (3.14 mg, 0.018 mmol) The mixture was stirred at RT for 24h, then concentrated under reduced pressure and the residue was purified by HPLC semi-preparative X-Terra RP-18, eluting with a linear gradient H₂O/MeCN (85:15 to 70:30), to give **68** (5 mg, 40%).
**1H NMR (500MHz, CDCl₃)** δ 7.60 (dd, 1H), 7.28 (s, 1H), 7.22-7.15 (m, 1H), 7.0 (m, 1H), 6.90 (d, 1H), 6.85-6.80 (1H), 6.65 (s, 1H), 6.60 (s, 1H), 6.33 (s, 1H), 6.15 (s, 1H), 4.20 (t, 2H), 4.05 (m, 1H), 3.78 (s, 3H), 3.49 (s, 6H), 3.38-3.33 (m, 1H), 3.25 (s br, 1H), 3.21-3.16 (m, 1H), 2.99-2.85 (m. 4H), 2.81-2.67 (m, 4 H), 2.63-2.48 (m. 11H), 2.41 (s, 4H), 2.30 (s, 3H), 2.14-2.07 (m, 2H).
**¹³C NMR (125MHz, CDCl₃)** δ 154.3, 150.9, 146.6, 146.2, 139.9, 139.8, 139.6, 139.5, 135.5, 135.4, 131.3, 130.0, 129.7, 128.5, 127.5, 122.8, 121.8, 120.7, 117.6, 115.8, 114.6, 114.2, 106.7, 60.57, 56.4, 55.3, 55.2, 53.3, 50.5, 46.2, 45.3, 42.9, 42.1, 42.1,41.5, 27.8, 27.0, 23.8.
**MS-ESI** (m/z) calculated for C₄₃H₅₁N₄O₅ [M+H]⁺: 703.2; Found: 703.4.

### Synthesis of 60

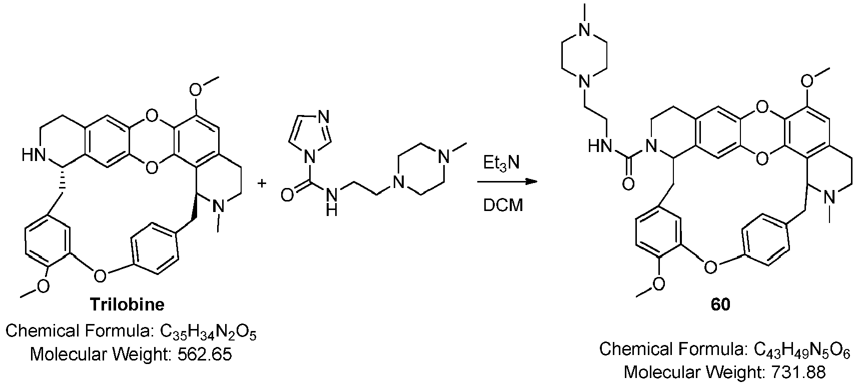

A solution containing 1-(2-aminoethyl)-4-methylpiperazine (4µL - 27 (µmol) and (500µL), 1,1carbonyldimidazole (4.5 mg - 28 (µmol) in DCM was stirred at RT for 2h. Next *O*-methylcocsoline (5.0 mg - 8.9 (µmol) was added at solution. The reaction mixture was stirred in at RT for 12h, then concentrated under reduced pressure and the residue was purified by HPLC semi-preparative X-Terra RP-18, eluting with a linear gradient H₂O/MeCN with (65:35 to 0:100), to give **60** (1.2 mg, 18%).
**¹H NMR (500MHz, CDCl₃)** δ 7.65-7.63 (m, 1H), 7.28-7.7.20 (m, 1H), 6.88-6.85 (m, 1H), 6.81-6.79 (m, 1H), 6.68-6.66 (m, 1H), 6.33 (s, 1H), 6.10 (s, 1H), 4.65-4.60 (m, 1H), 4.48-4.45 (m, 1H), 4.42-4.39 (m, 1H), 4.08-4.05 (m, 1H), 3.97 (s, 3H), 3.87 (s, 3H), 3.39-3.33 (m, 1H), 3.30-3.26 (m,1H), 3.21-3.12 (m, 2H), 3.08-3.04 (m, 1H), 2.98-2.90 (m, 3H), 2.85-2.80 (m, 1H), 2.75-2.70 (m, 2H), 2.62 (s, 3H), 2.55-2.48 (m, 2H), 2.35-2.29 (m, 2H), 2.26 (s, 3H).
**MS-ESI** (m/z) calculated for C₄₃H₅₀N₅O₆ [M+H]⁺: 732.8; Found: 732.4.

### Synthesis of 48

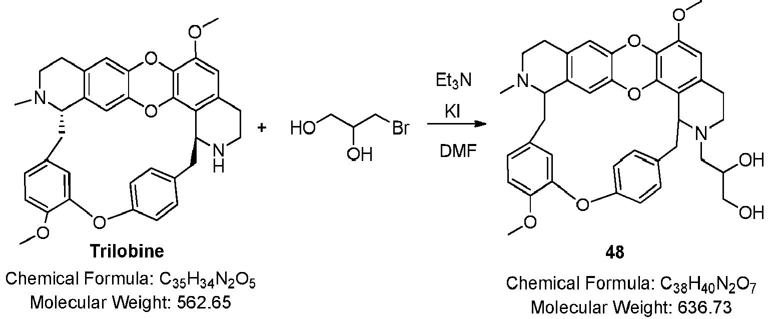

To a stirred solution of Trilobine (5 mg - 8.9 (µmol) in DMF (500 µL), 3-bromopropane-1,2-diol (4.0 µL, 46 µmol), KI (1 mg, 6.0 (µmol) and Et₃N (4.0 µL, 30 (µmol) were added and the reaction mixture was stirred at 65°C for 24h. The reaction mixture was concentrated under reduced pressure and the residue was purified by HPLC semi-preparative X-Terra RP-18, eluting with a linear gradient H₂O/MeCN (80:20 to 0:100), to give **48** (2.0 mg, 38%).
**¹H NMR (500MHz, CDCl₃)** δ 7.63-7.50 (m, 1H), 7.29-2.20 (m, 4H), 7.18-7.11 (m, 2H), 7.00-6.95 (m, 1H), 6.88-6.82 (m, 3H), 6.60-6.53 (m, 2H), 6.30-3.28 (m, 1H), 6.14-6.12 (m, 1H), 4.11-4.09 (m, 1H), 4.08-3.98 (m, 2H), 3.97 (s, 3H), 3.94-3.92 (m, 1H), 3.88 (s, 3H), 3.82-3.78 (m, 1H), 3.65-3.64 (m, 1H), 3.62-3.59 (m, 1H), 3.38-3.35 (m, 1H), 3.25-3.23 (m, 2H), 3.11-2.42 (m, 15 H), 2.40 (s, 3H).
**MS-ESI** (m/z) calculated for C₃₈H₄₁N₂O₇ [M+H]⁺: 637.7; Found: 637.2.

### Synthesis of 47

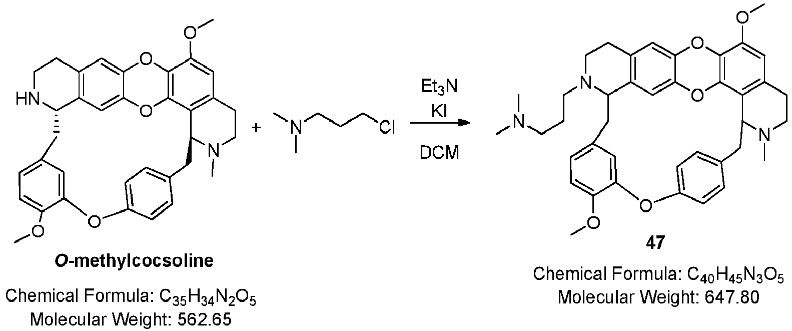

To a stirred solution containing *O*-methylcocsoline (5.0 mg, 8.9 (µmol) in DMF (500µL), 3-chloro-*N,N*dim6thylpropan-1-amine chloride (7.0 mg, 44.0 µmol), KI (1.0 mg, 6.0 (µmol) and triethylamine (10.0 µL - 75.0 (µmol) were added and the reaction mixture was stirred at 65°C for 24h. The reaction mixture was concentrated under reduced pressure and the residue was purified by HPLC semi-preparative X-Terra RP-18, eluting with a linear gradient H₂O/MeCN (80:20 to 0:100), to give **47** (1.2 mg, 21%).
**¹H NMR (500MHz, CDCl₃)** δ 7.61-7.58 (m, 1H), 7.27-7.25 (m, 1H), 7.18-7.15 (m, 1H), 7.02-6.98 (m, 1H), 6.85-6.79 (m, 2H), 6.60-6.58 (m, 1H), 6.39 (s, 1H), 6.07 (s, 1H), 4.05-4.03 (m, 1H), 3.97 (s, 3H), 3.88 (s, 3H), 3.49 (s, 3H), 3.48-3.45 (m, 1H), 3.42-3.39 (m, 1H), 3.28-3.20 (m, 3H), 2.92-2.85 (m, 5H), 2.75-2.60 (m, 5 H), 2.58 (s, 3H), 2.52-2.48 (m, 3H), 2.92-2.85 (m, 2H), 2.25-2.22 (m, 2H), 2.18 (s, 3H).
**MS-ESI** (m/z) calculated for C₄₀H₄₆N₃O₅ [M+H]⁺: 648.8; Found: 648.3.

### Synthesis of 42

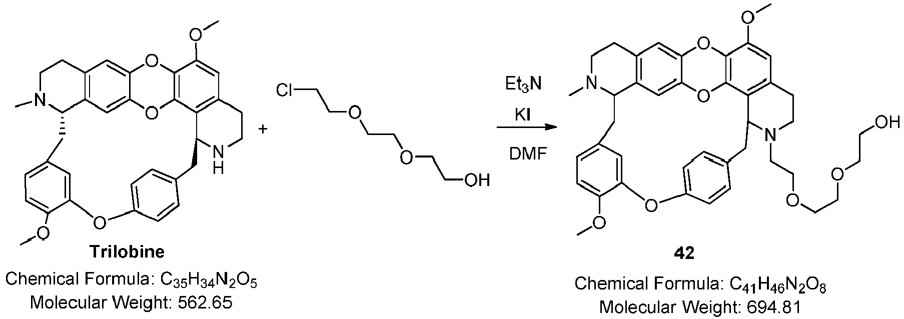

To a stirred solution containing Trilobine (5.0 mg, 8.9 µmol) in DMF (500 µL), 2-(2-(2-chloroethoxy)ethoxy)ethanol (3.0µL, 21 µmol), KI (1.0 mg - 6.0 µmol) and Et3N (4.0 µL - 30 µmol) were added and the reaction mixture was stirred at 65°C for 24h. The reaction mixture was purified by HPLC semi-preparative X-Terra RP-18, eluting with a linear gradient H₂O/MeCN with 0.02% TEA (80.20 to 0:100), to give **42** (1.2 mg, 1.7 µmol, 19%).
**HRMS-ESI** (m/z) calculated for C₄₁H₄₆N₂O₈Na [M+Na]⁺: 717.3146; Found: 717.3142.

### Synthesis of 43

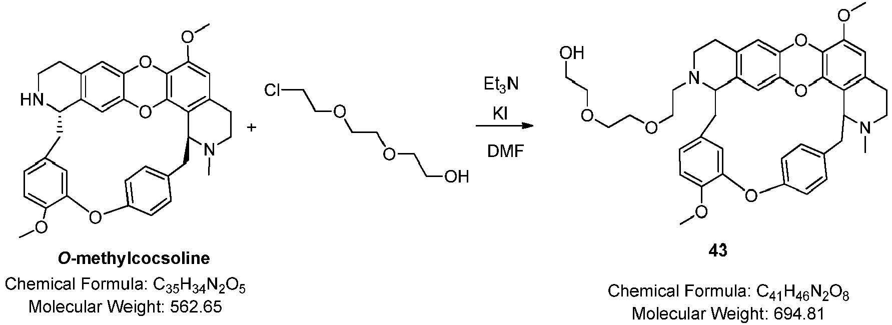

To a stirred solution containing *O*-methylcocsoline (5.0 mg - 8.9 µmol) in DMF (500 µL), 2-(2-(2-chloroethoxy)ethoxy)ethanol (3.0 µL - 21.0 µmol), KI (1.0 mg - 6.0 µmol) and Et3N (4.0 µL - 30 µmol) were added and the reaction mixture was stirred at 65°C for 24h. The reaction mixture was purified by HPLC semi-preparative X-Terra RP-18, eluting with a linear gradient H₂O/MeCN with 0.02% TEA (80.20 to 0:100), to give **43** (1.7 mg, 2.4 µmol, 27%).
**MS-ESI** (m/z) calculated for C₄₁H₄₇N₂O₈ [M+H]⁺: 695.81; Found: 695.1.

### Synthesis of 28

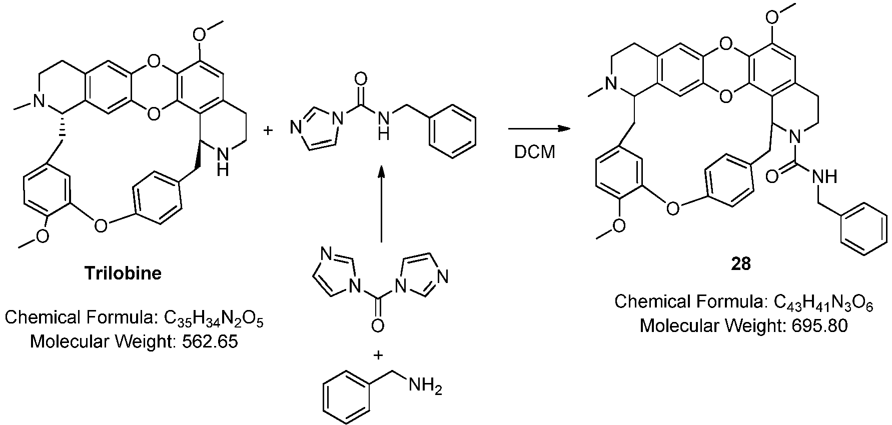

A solution containing pyridine-4-methanamine (3.1 µL, 28.0 µmol) and 1,1carbonyldimidazole (4.5 mg - 28.0 µmol) in DCM (500µL), was stirred at RT for 2 h. Next, Trilobine (5.0 mg, 8.9 µmol) was added to the solution. The reaction mixture was stirred at RT for 12 h., then purified by HPLC semi-preparative X-Terra RP-18, eluting with a linear gradient H₂O/MeCN with 0.02% TEA (80:20 to 0:100), to give **28** (1.6 mg, 2.3 µmol, 26%).
**HRMS-ESI** (m/z) calculated for C₄₂H₄₁N₄O₆ [M+H]⁺: 696.3068; Found: 696.3047.

### Synthesis of 31

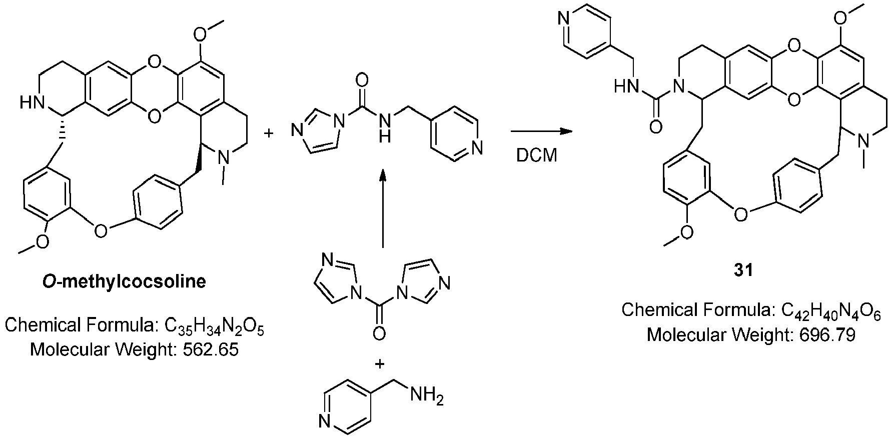

A stirred solution containing pyridine-4-methanamine (2.9 µL, 29.0 µmol) and 1,1carbonyldimidazole (4.5 mg - 28.0 µmol in DCM (500µL),) was stirred at RT for 2 h. Next, *O*-methylcocsoline (5.0 mg, 8.9 µmol) was added to the solution. The reaction mixture was stirred at RT for 48 h. The reaction mixture was purified by HPLC semi-preparative X-Terra RP-18, eluting with a linear gradient H₂O/MeCN with 0.02% TEA (65:35 to 15:85), to give **31** (1.1 mg, 1.6 µmol, 17%).
**¹H NMR (500MHz, CDCl₃)** δ 8.83-8.51 (m, 4H), 7.64-7.61(m, 2H), 7.23-7.21 (m, 1H), 7.19-7.18 (m, 1H), 7.08-7.00 (m, 2H), 6.65-6.55 (m, 3H), 6.41 (s, 1H), 6.05 (s, 1H), 4.48-4.41 (m, 2H), 4.38-4.30 (m, 2H), 4.22-4.18 (m, 2H), 4.08-4.05 (m ,1H), 3.89 (s, 3H), 3.85 (s, 3H), 3.49 (s br, 1H), 3.38-3.35 (m, 2H), 3.19-3.08 (m, 3H), 2.98-2.85 (m, 3H) 2.82-2.68 (m, 4H), 2.39 (s, 3H), 2.55-2.45 (m, 3H).
**HRMS-ESI** (m/z) calculated for C₄₂H₄₁N₄O₆ [M+H]⁺: 697.3021; Found: 697.3328.

### Synthesis of 23

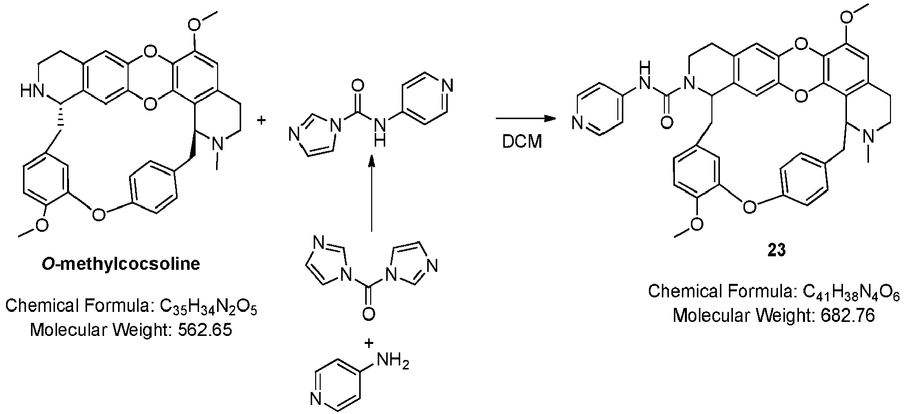

A solution containing pyridine-4-amine (2.7 mg, 29 µmol) in DCM (500 µL), 1,1carbonyldimidazole (4.5 mg - 28 µmol) was stirred at RT for 2 h. Next, O-methylcocsoline (5.0 mg, 8.9 µmol) was added to the solution. The reaction mixture was stirred at RT for 48 h. The reaction mixture was purified by HPLC semi-preparative X-Terra RP-18, eluting with a linear gradient H₂O/MeCN with 0.02% TEA (65.35 to 15:85), to give **23** (2.6 mg, 3.8 µmol, 43%).
**HRMS-ESI** (m/z) calculated for C₄₁H₃₉N₄O₆ [M+H]⁺: 682.2864; Found: 683.2854.

### Synthesis of 27

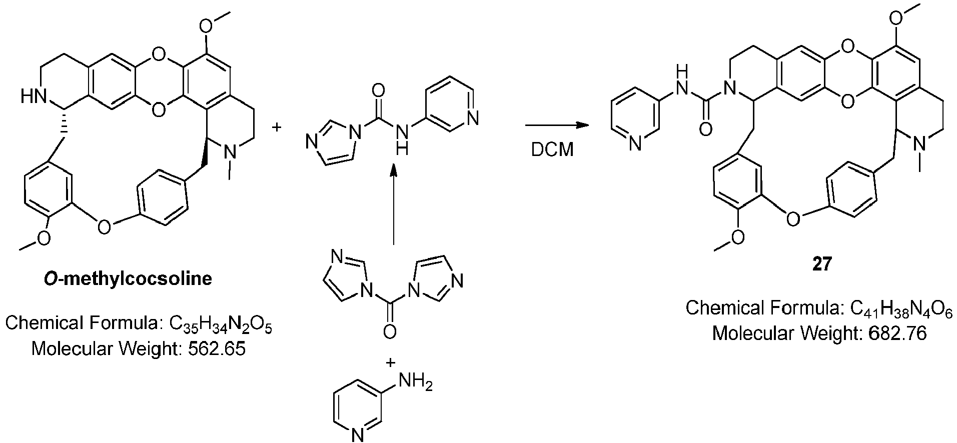

A solution containing 3-amino-pyridin (2.7 mg - 29.0 µmol) in DCM (500 µL), 1,1carbonyldimidazole (4.5 mg - 28.0 µmol) was stirred at RT for 2 h. Next, O-methylcocsoline (5.0 mg - 8.9 µmol) was added to the solution. The reaction mixture was stirred at RT for 48h, then purified by HPLC semi-preparative X-Terra RP-18, eluting with a linear gradient H₂O/MeCN with 0.02% TEA (65.35 to 15.85), to give **27** (1.7 mg, 2.5 µmol, 28%).
**HRMS-ESI** (m/z) calculated for C₄₁H₃₉N₄O₆ [M+H]⁺: 683.2864; Found: 683.2853.

### Example 1:Inhibition activity of the proliferation of asynchronous asexual cultures

### Materials and methods:

Compounds were provided at a concentration of 10 mM in DMSO.

*P. falciparum* parasite are cultured using standard protocol (Trager and Jensen, Science 1976, 193(4254), 673-675). Briefly, parasites are cultured at 4% hematocrit (O+ human blood, Etablissement Français du Sang, France) in RPMI1640 complemented with 0.1% Albumax I, HEPES buffer, hypoxanthine, and gentamycin. They are incubated at 37°C under an atmosphere of 5% CO2 and 5% O2.

Compounds of the invention were assayed for a first screen at 5µM in duplicate against asynchronous asexual cultures of *P. falciparum* NF54 (drug-sensitive line) containing mostly rings (2% hematocrit. 0.5% starting parasitemia). Plates were incubated for 72h at 37°C under a reduced oxygen atmosphere. Parasite growth was assessed using the SYBR Green I based assay.

Compounds inducing less than 40% survival at 5µM were assayed for IC50 in duplicate using a 7-concentration range with 3-fold dilutions, in the same conditions. For the most active compounds, three independent experiments were realized using a range with 2-fold dilutions. IC50 were determined using Graphpad Prism 6 software. Dihydroartemisinin (starting concentration 50nM) and DMSO were used as positive and negative controls.

### Results:

**Table 1 - Inhibition activity of the proliferation of asynchronous asexual cultures of P.falciparum NF54.**

| entry | Structure | % survival @5uM | Malaria CI₅₀ nM |
|---|---|---|---|
| 10 | 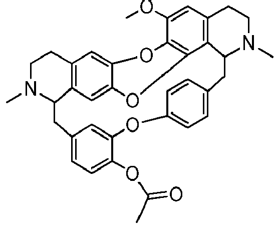 | 11,7 | 470+/-62 |
| 23 | 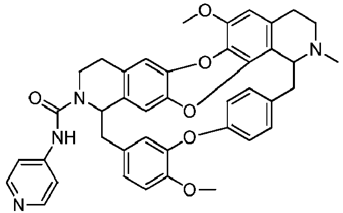 | 13,4 | 223+/-58 |
| 27 | 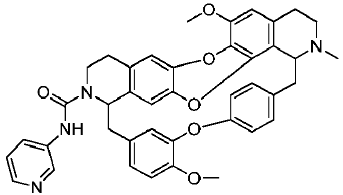 | 12,7 | 383+/-173 |
| 28 | 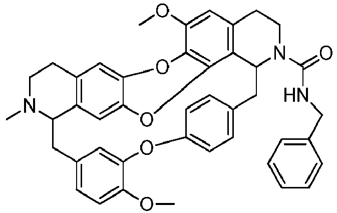 | 14,4 | 504+/-134 |
| 31 | 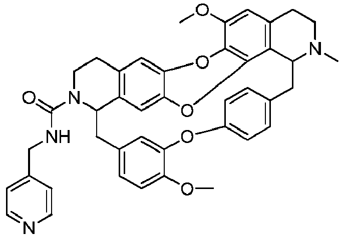 | 14,8 | 151+/-21 |
| 42 | 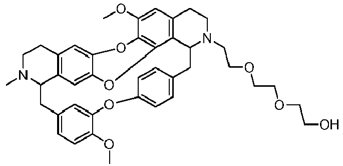 | 12,9 | 437+/-83 |
| 43 | 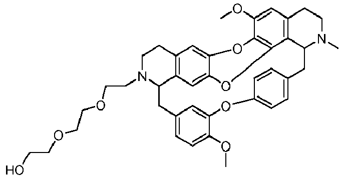 | 13,3 | 438+/-95 |
| 46 | 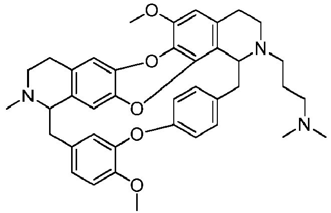 | 14,2 | 169+/-10 |
| 48 | 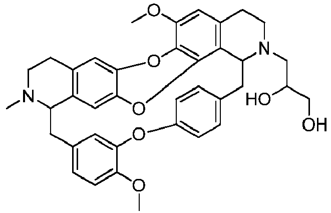 | 11,8 | 195+/-55 |
| 60 | 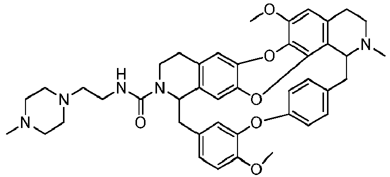 | 15,4 | 227+/-59 |
| 68 | 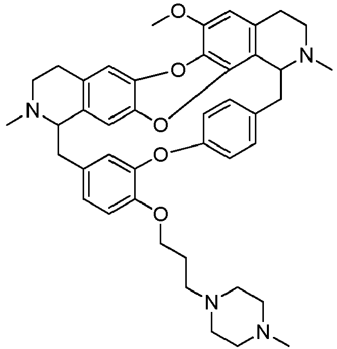 | 14,8 | 189 |
| 73 | 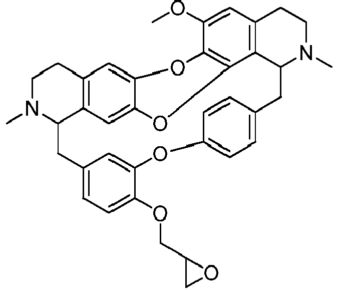 | 15,8 | 299+/-25 |
| 74 | 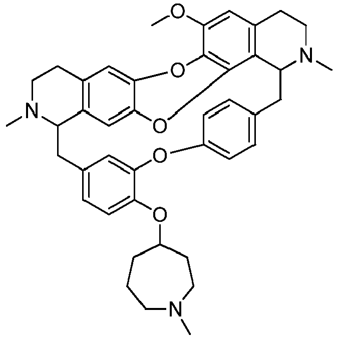 | 14,7 | 297+/-42 |
| 75 | 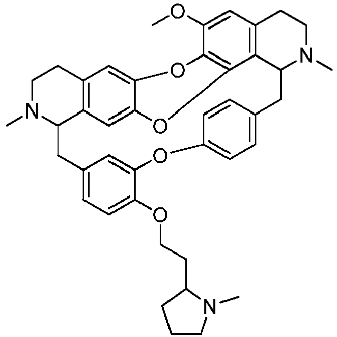 | 14,9 | 352+/-54 |
| 78 | 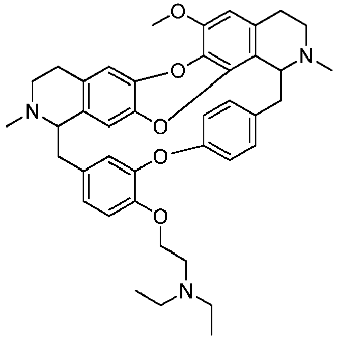 | 12,6 | 331+/-29 |
| 82 | 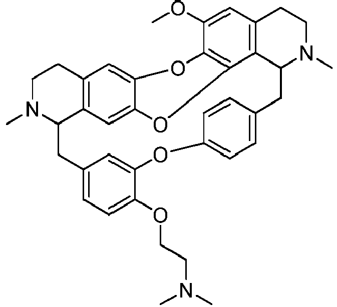 | 13,4 | 260+/-34 |
| 84 | 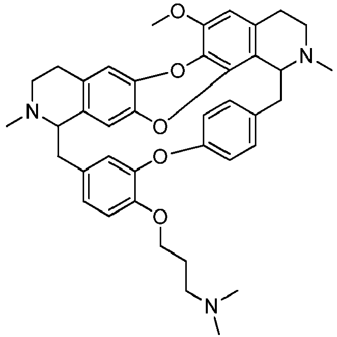 | 14,9 | 130+/-35 137 |
| 87 | 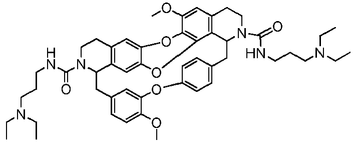 | 11,3 | 441+/-67 |
| 88 | 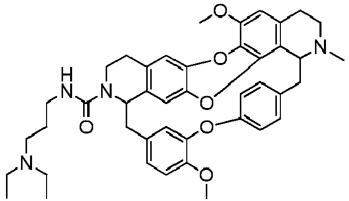 | 14,7 | 297+/-31 |
| 89 | 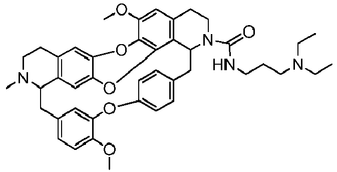 | 14,4 | 428+/-77 |
| 110 | 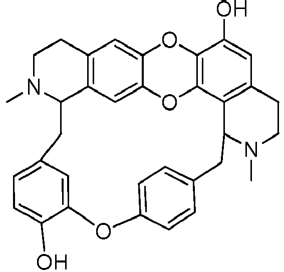 | | 146+/-39 |
| 113 | 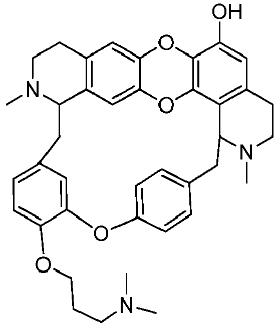 | | 153+/-58 |
| 116 | 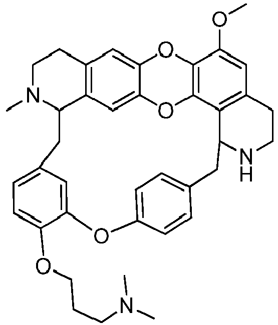 | | 263+/-50 |
| 121 | 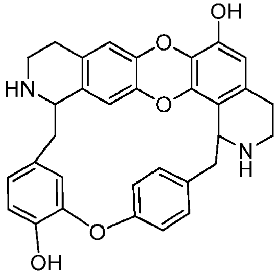 | | 115+/-55 |
| 122 | 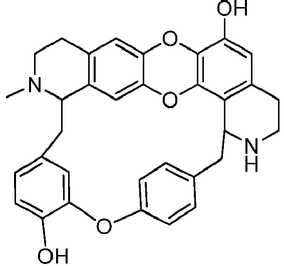 | | 248+/-58 |
| 123 | 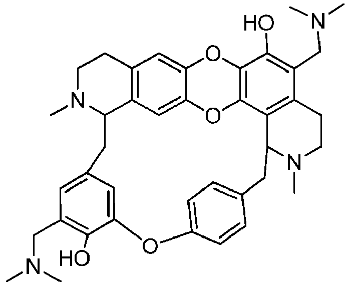 | | 283+/-155 |
| 124 | 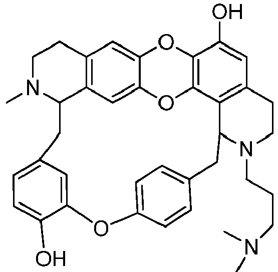 | | 423+/-239 |

The following results were also obtained, in the same conditions than the ones described above:

| | mean IC₅₀ +/- SD (nM) |
|---|---|
| Nortrilobine | 1220 ± 130 |
| Isotrilobine | 1775 |

### Example 2: Effect of compounds of the invention against Cambodian multi-resistant strains.

Compounds 63 and 84 were evaluated regarding the inhibition of the proliferation of multi-resistant field isolates adapted to culture. A panel of four Cambodian strains bearing different molecular markers of resistance were selected: **3D7:** Laboratory strain, drug-sensitive, **5150:** Cambodian isolate resistant to Chloroquine and Pyrimethamine, **6591:** Cambodian isolate resistant to Chloroquine, Pyrimethamine, **5248:** Cambodian isolate resistant to Chloroquine, Pyrimethamine, Mefloquine and Artemisinin, **6320:** Cambodian isolate resistant to Chloroquine, Pyrimethamine, Piperaquine and Artemisinin.

IC₅₀ were obtained against asynchronous asexual stages using SYBR-green assay after 72h of incubation with a serial dilution of the compounds. Data represents the mean and SD of 3 independent experiments ran in triplicates. Table 2 illustrates mean IC₅₀ values and their standard deviation in nanomolar.

**Table 2**

| IC50 nM | Strain 6591 | | Strain 5248 | | Strain 5150 | | Strain 6320 | | Lab. Strain 3D7 | |
|---|---|---|---|---|---|---|---|---|---|---|
| | mean* | SD | mean* | SD | mean* | SD | mean* | SD | mean* | SD |
| 84 | 167 | 27 | 346 | 165 | 251 | 74 | 183 | 8 | 136 | 42 |
| Pyrimethamine | >5000 | | >5000 | | >5000 | | >5000 | | 30 | 4 |
| Chloroquine | 64 | 5 | 98 | 20 | 55 | 9 | 109 | 3 | 13 | 6 |
| Piperaquine | 34 | 2 | 33 | 9 | 26 | 5 | >200 | | 41 | 17 |

| | | | | | | | | | | |
|---|---|---|---|---|---|---|---|---|---|---|
| * mean value of three replicates | | | | | | | | | | |

### Example 3: Stage-specificity during the asexual cell cycle.

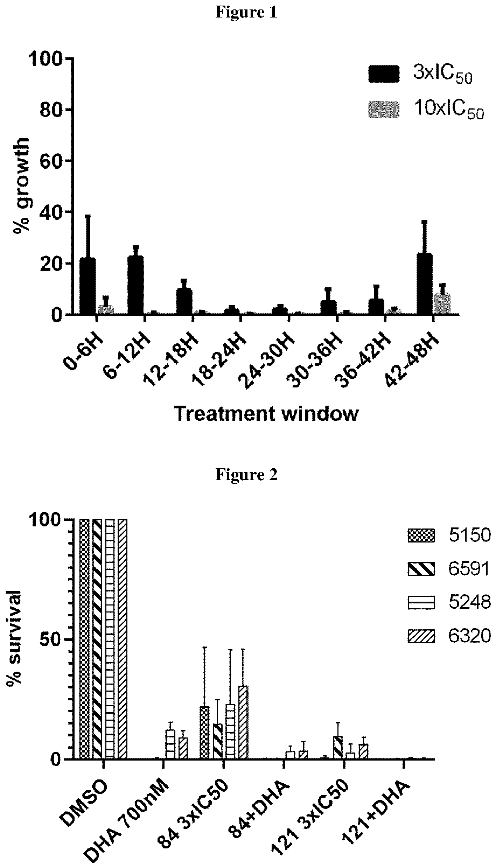

To assess stage specificity of compounds of the invention, asexual NF54 parasites were tightly synchronized (0 to 3h post-invasion) using gelatin flotation and 5% D-sorbitol-treatment 3h later. These parasites were then dispensed in 48-well plates (500µL per well at 2% hematocrit, 0.5% starting parasitemia) and incubated for six hours with different concentration of the compounds (corresponding to one time, two times and three times the IC₅₀ value), either directly (0-6h treatment window) or after 6h, 12h, 18h, 24h, 30h, 36h, or 42h. Following the 6h-treatment, the corresponding cells were pelleted, washed with 10ml RPMI, and put back into culture in 500uL of complete media in a new plate. Parasitemia was assessed at 72 hours post-synchronization using giemsa-stained thin blood smears. Percentage of survival was calculated in comparison to a DMSO-treated control. The data were obtained in three independent experiments. Compound 84 is active throughout the 48h cycle with an increased efficiency between 12 and 42h post-invasion (Figure 1). This compound is active starting from the concentration corresponding to 3 times the IC₅₀ value.

### Example 4: Ring-stage Survival Assay (RSA).

Since compounds of the invention are active on early ring stage as shown by the experiments on the cell cycle, their ability to kill artemisinin-resistant parasites in the ring-stage survival assay was tested (strains 5150, 6591, 5248 and 6320, Figure 2).

0-3h synchronized ring-stage parasites were exposed to a treatment of 6h with either DMSO, 700nM dihydroartemisinin (DHA), three times the IC₅₀ value of the compound to test or 700nM DHA in combination with three times the IC₅₀ value of the compound to test. Parasitemia was assessed at 72 hours post-synchronization using giemsa-stained thin blood smears. Percentage of survival was calculated in comparison to a DMSO-treated control. The data were obtained in three independent experiments.

Compound 84 kills resistant rings using three times the IC₅₀ value in a similar manner whichever the strain considered, as observed on sensitive cell line NF54 (figure 2, 0-6h treatment window). This compound is able to kill the parasites of all the tested strains in combination with 700nM dihydroartemisinin (DHA).

### Example 5: Evaluation of the parasite reduction rate (time needed for the compounds of the invention to kill the parasites).

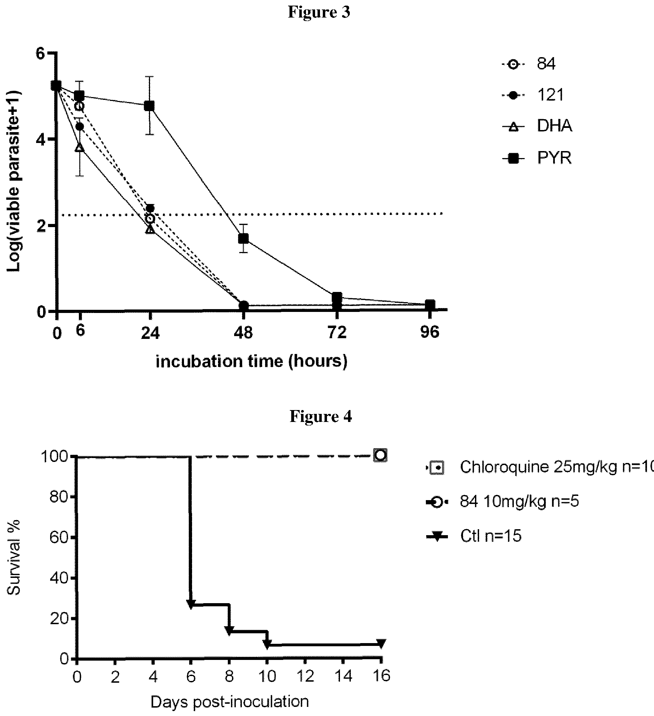

Parasites are incubated (see for example Sanz et al. PLoS ONE 7, e30949 (2012)) with a compound of the invention (or a reference) for different time-windows, washed, and diluted serially in 96-well plates in fresh red blood cells. Parasites are kept in culture for 3 to 4 weeks, when growth is assessed to evaluate the number of parasites that survive-correlated to the number of wells where growth is found. Survival curves are obtained by plotting the duration of treatment versus the log of the number of viable parasites (figure 3). Compounds 84 and 121 of the invention act as fast as dihydroartemisinin (DHA), which is the fastest killer described so far, whereas pyrimethamine (PYR) is known as a slow killer.

### Example 6: In vivo activity

*In vivo* activity was determined as previously described (Malmquist et al. Antimicrob. Agents Chemother. 2015, 59 (2), 950-959) following the Peters 4-day suppressive test (Peters, Ann. Trop. Med. Parasitol. 1975, 69 (2), 155-171), with slight modifications. C57BL/6 mice were infected intraperitoneally (i.p.) with 10⁵ *P. berghei* ANKA strain GFP-expressing parasites (Ishino et al. Cell. Mol. Microbiol. 2006, 59 (4), 1175-1184). Two hours post-infection, mice were treated i.p. with a daily regimen of 10mg/kg of compound 84, or 25mg/kg of chloroquine, or the equivalent vehicle control (distillated water) for four days. Parasitemia was quantified from blood samples collected every day by flow cytometry of 50,000 RBCs and confirmed by Giemsa-stained blood smears. When treated with compound 84 at 10mg/kg, the mice do not develop severe malaria symptoms and are able to survive until day 16 after inoculation.

## Claims

1. A compound of following formula (I) 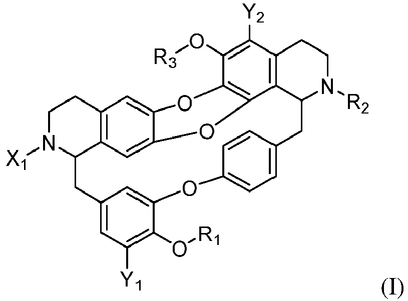 Wherein,
Y₁ and Y₂ are each independently H or (C₁-C₁₀)-alkyl, in which the fragments (C₁-C₁₀)-alkyl are optionally substituted by at least one group selected from NR_{Y1}R_{Y2};
R_{Y1} and R_{Y2} are each independently H or (C₁-C₁₀)-alkyl;
X₁ is H, methyl, ethyl, (C₁-C₁₀)-alkyl-R₄, (C₂-C₁₀)-alkenyl, (C₂-C₁₀)-alkenyl-R₄, CONHR₅, (CH₂-CH₂O)ₘ-H with m=2-4, in which the fragments (C₁-C₁₀)-alkyl are optionally substituted by at least one group selected from halogens;
and N-X₁ optionally represents N⁺-O⁻;
R₂ is chosen from H, methyl, ethyl, (C₁-C₁₀)-alkyl-R_{4'}, (C₂-C₁₀)-alkenyl, (C₂-C₁₀)-alkenyl-R_{4'}, CONHR_{5'}, (CH₂-CH₂O)ₘ-H with m=2-4, (C₁-C₁₀)-alkyl substituted by at least one group selected from halogen, -OH and -O-(C₁-C₁₀)-alkyl;
R₄ is OH, O-(C₁-C₁₀)-alkyl, O-(C₅-C₁₀)-aryl, NO₂, CN, (3-12)-membered-heterocycle, O-((CH₂)₂O)ₙ-H with n=1-3, CONR_{c}R_{d}, halogen, COOR_{c}, CF3, or (C₃-C₁₂)-cycloalkyl, in which (C₅-C₁₀)-aryl and (3-12)-membered-heterocycle are optionally substituted by at least one group selected from NR_{c}R_{d}, ORₑ, (C₁-C₁₀)-alkyl, halogen and oxo (=O);
R₅ is H, or a group chosen among (C₁-C₁₀)-alkyl-R₆, (C₃-C₁₂)-cycloalkyl, (C₂-C₁₀)-alkenyl, and a (3-12)-membered-heteroaryl, said group being optionally substituted by at least one group selected from halogen, CN, NR_{c}R_{d}, NO₂, CONR_{c}R_{d}, COOR_{c}, CF3, ORe,
R_{4'} is OH, O-(C₁-C₁₀)-alkyl, O-(C₅-C₁₀)-aryl, NO₂, CN, NR_{c}R_{d}, (3-12)-membered-heterocycle, O-((CH₂)₂O)ₙ-H with n=1-3, CONR_{c}R_{d}, halogen, COOR_{c}, CF3, or (C3-C₁₂)-cycloalkyl, in which (C₅-C₁₀)-aryl and (3-12)-membered-heterocycle are optionally substituted by at least one group selected from NR_{c}R_{d}, OR_{c}, (C₁-C₁₀)-alkyl, halogen and oxo (=O);
R_{5'} is H, or a group chosen among (C₁-C₁₀)-alkyl-R_{6'}, (C₃-C₁₂)-cycloalkyl, (C₂-C₁₀)-alkenyl, benzyl, and, only when R₁ and X₁ are not H, Me, said groups being optionally substituted by at least one group selected from halogen, CN, NR_{c}R_{d}, NO₂, CONR_{c}R_{d}, COOR_{c}, CF₃, ORₑ;
R6 is NR_{c}R_{d}, (3-12)-membered-heteroaryl or (3-12)-membered-heterocycle, in which the fragment (3-12)-membered-heteroaryl or (3-12)-membered-heterocycle is optionally substituted by at least one group selected from NR_{c}R_{d}, ORₑ, (C₁-C₁₀)-alkyl, (C₁-C₁₀)-alkyl-(3-12)-membered-heterocycle, halogen and oxo;
R_{6'} is NR_{c}R_{d} or (3-12)-membered-heterocycle, in which the (3-12)-membered-heterocycle is optionally substituted by at least one group selected from (C₁-C₁₀)-alkyl;
R₁ is H, methyl, -(C₁-C₆) alkyl-NRaRb, COR₇, (3-12)-membered-azaheterocycle optionally substituted by at least one group selected from (C₁-C₁₀)-alkyl, or (C₁-C₆)-alkyl substituted by at least one group selected from epoxide and (3-12)-membered-azaheterocycle, both optionally substituted by at least one group selected from (C₁-C₁₀)-alkyl,
Ra and Rb are independently from each other (C₁-C₆) alkyl or H, at least one of Ra and R_{b} being a (C₁-C₆) alkyl;
R_{c}, Rd and Rₑ are for each occurence independently from each other (C₁-C₆) alkyl or H; R3 is H, (C₁-C₆) alkyl, (C₁-C₆) alkyl substituted by NR_{c}R_{d};
R₇ is (C₁-C₁₀)-alkyl, (C₅-C₁₀)-aryl;
With the proviso that:
- in case Y₁ and Y₂ are H, and R₁, R₂, R₃ and X₁ are independently chosen from H and methyl, then R₃ is H and R₂ is H;
or a pharmaceutically acceptable salt or solvate thereof,
for use in the treatment and/or prevention of malaria.

2. The compound for use according to claim 1, wherein it is a compound of one of the following formulae:

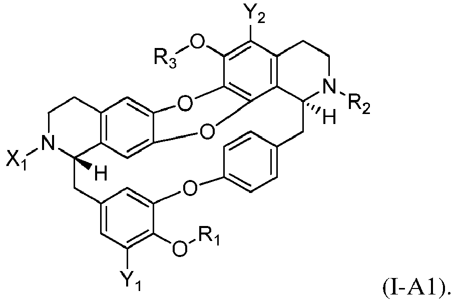

3. The compound for use according to anyone of claims 1 to 2, wherein :
- Y₁ and/or Y₂, in particular Y₁ and Y₂, are H; and/or
- R₃ is H or methyl, in particular methyl.

4. The compound for use according to anyone of claims 1 to 3, wherein :
- one of R₁, R₂ and X₁ is not H nor methyl, the two others being notably H or methyl;
- R₁ is not H nor methyl, R₂ and X₁ being in particular independently H or methyl;
- R₂ is not H nor methyl, R₁ and X₁ being in particular independently H or methyl;
- X₁ is not H nor methyl, R₁ and R₂ being in particular independently H or methyl; or
- X₁ and R₂ are not H nor methyl, X₁ and R₂ being in particular identical, R₁ being notably H or methyl.

5. The compound for use according to anyone of claims 1 to 3, of following formula (II) 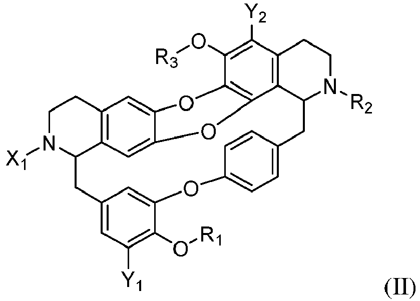 Wherein:
Y₁, Y₂ and R₃ are as defined in claim 1, Y₁ and Y₂, being in particular H, and R₃ being in particular methyl,
X₁ is H, methyl, CONHR₅, (CH₂-CH₂O)ₘ-H with m=2-4;
R2 is chosen from H, methyl, (C₁-C₁₀)-alkyl-R_{4'}, CONHR_{5'}, and (CH₂-CH₂O)ₘ-H with m=2-4, (C₁-C₁₀)-alkyl being optionally substituted by at least one -OH;
R₅ is a group chosen among (C₁-C₁₀)-alkyl-R₆, and (3-12)-membered-heteroaryl, in particular pyridyl;
R_{4'} is NR_{c}R_{d};
R_{5'} is a group chosen among (C₁-C₁₀)-alkyl-R_{6'}, benzyl, and, only when R₁ and X₁ are not H, Me;
R6 is NR_{c}R_{d}, (3-12)-membered-heteroaryl, in particular pyridyl, or (3-12)-membered-heterocycle, in particular piperazinyl, in which the fragment (3-12)-membered-heteroaryl or (3-12)-membered-heterocycle is optionally substituted by at least one group selected from (C₁-C₁₀)-alkyl;
R_{6'} is NR_{c}R_{d};
R₁ is H, methyl, -(C₁-C₆)alkyl-NRₐR_{b}, COR₇, (3-12)-membered-azaheterocycle, in particular azepanyl, optionally substituted by at least one group selected from (C₁-C₁₀)-alkyl, or (C₁-C₆)-alkyl substituted by at least one group selected from epoxide and (3-12)-membered-azaheterocycle, in particular pyrrolidinyl or piperazinyl, both epoxide and (3-12)-membered-azaheterocycle being optionally substituted by at least one group selected from (C₁-C₁₀)-alkyl,
Ra and R_{b} are independently from each other (C₁-C₆) alkyl or H, at least one of Ra and R_{b} being a (C₁-C₆) alkyl;
R_{c}, Rd and Rₑ are for each occurence independently from each other (C₁-C₆) alkyl or H;
R₇ is (C₁-C₁₀)-alkyl, (C₅-C₁₀)-aryl;
with the proviso that:
- in case R₁, R₂, R₃ and X₁ are independently chosen from H and methyl, then R₃ is H and R₂ is H.

6. The compound for use according to anyone of claims 1 to 5, wherein in case R₁, R₂, R₃ and X₁ are independently chosen from H and methyl, then R₃ is H, and R₂ is H, R₁ being in particular H.

7. The compound for use according to claim 1, wherein it is selected from the following compounds:

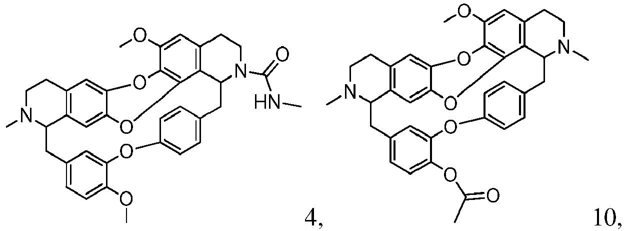

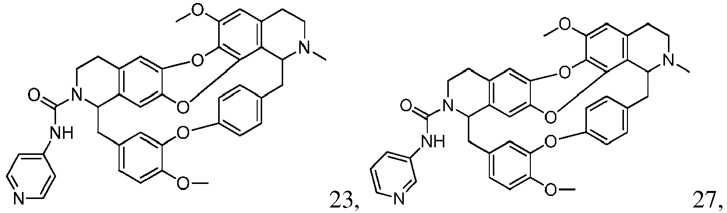

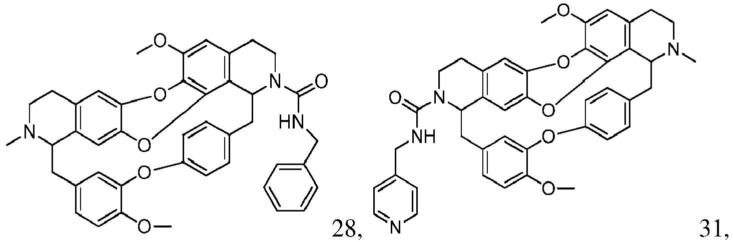

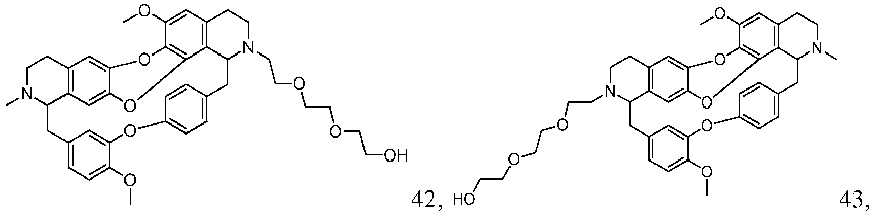

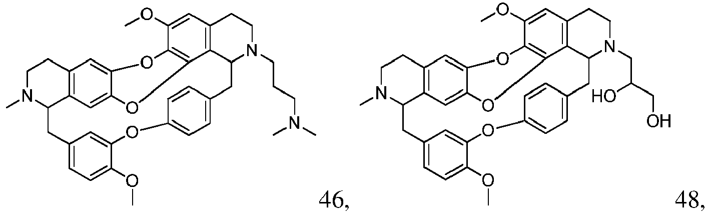

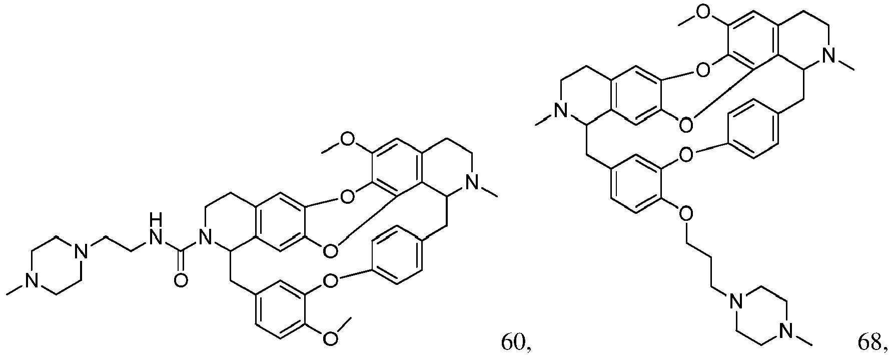

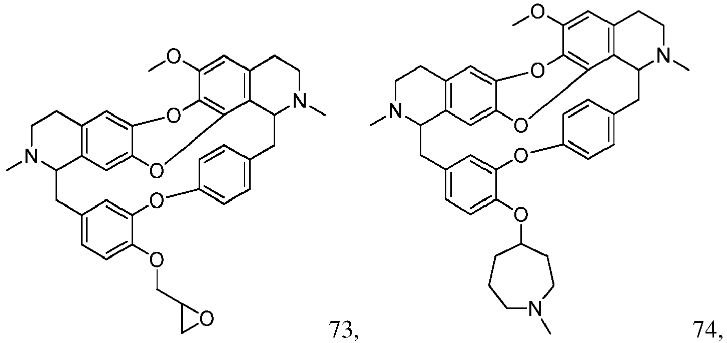

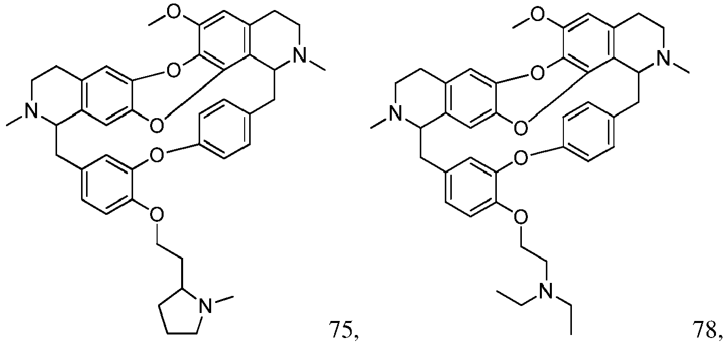

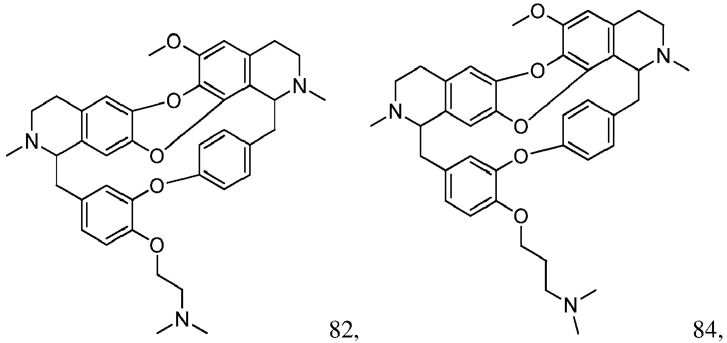

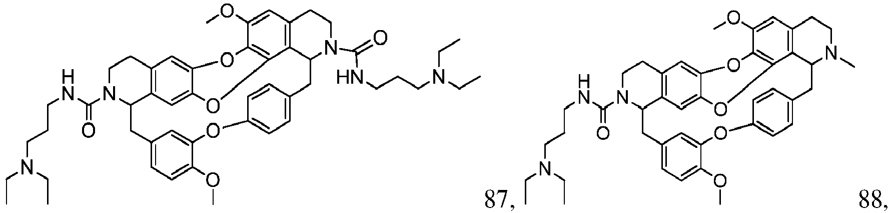

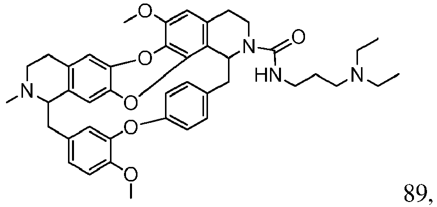

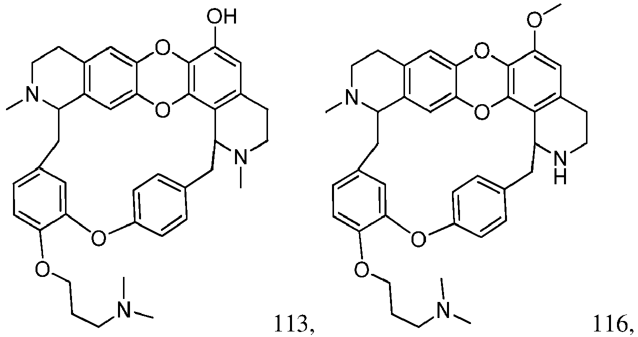

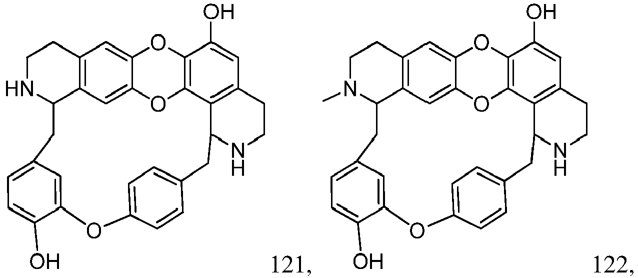

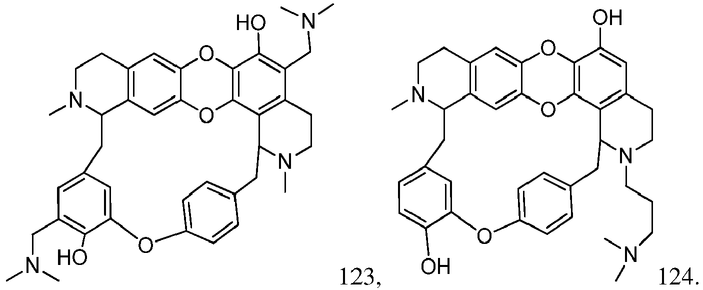

8. The compound for use according to any one of claims 1 to 7, wherein the malaria is caused by a drug sensitive or drug resistant malaria parasite, in particular a drug resistant malaria parasite.

9. The compound for use according to claim 8, wherein the drug-sensitive or drug-resistant malaria parasite is a chloroquine-sensitive; a chloroquine-resistant; a chloroquine and pyrimethamine-sensitive; a chloroquine and pyrimethamine-resistant; a chloroquine, pyrimethamine and mefloquine-sensitive; a chloroquine, pyrimethamine and mefloquine-resistant; a chloroquine, pyrimethamine, mefloquine and artemisinin-sensitive; a chloroquine, pyrimethamine, mefloquine and artemisinin-resistant; a chloroquine, pyrimethamine, piperaquine and artemisinin-sensitive; a chloroquine, pyrimethamine, piperaquine and artemisinin-resistant malaria parasite.

10. The compound for use according to claim any one of claims 1 to 9, wherein malaria is caused by infection with Plasmodium, in particular by *Plasmodium falciparum, P. vivax, P. ovale,* or *P. malariae, P. knowlesi* more particularly by *Plasmodium falciparum.*

11. A combination of a compound according to any one of claims 1 to 7, with at least one other antimalarial agent for use in the treatment of malaria with simultaneous administration, separate or spread out over time.

12. The combination for use according to claim 11, wherein the other antimalarial agent is selected from chloroquine, artemesin, qinghaosu, 8-aminoquinoline, amodiaquine, arteether, artemether, artemisinin, artesunate, artesunic acid, artelinic acid, atovaquone, azithromycine, biguanide, chloroquine phosphate, chlorproguanil, cycloguanil, dapsone, desbutyl halofantrine, desipramine, doxycycline, dihydrofolate reductase inhibitors, dipyridamole, halofantrine, haloperidol, hydroxychloroquine sulfate, imipramine, mefloquine, penfluridol, phospholipid inhibitors, primaquine, proguanil, pyrimethamine, pyronaridine, quinine, quinidine, quinacrineartemisinin, sulfonamides, sulfones, sulfadoxine, sulfalene, tafenoquine, tetracycline, tetrandine, triazine, salts and mixture thereof.

13. A pharmaceutical composition comprising a compound of following formula (III): 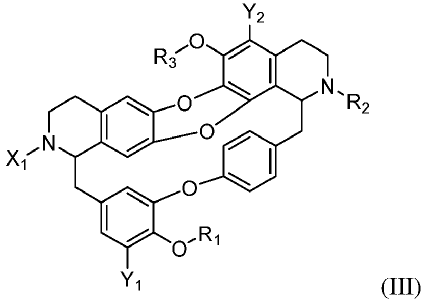 Wherein,
Y₁ and Y₂ are each independently H or (C₁-C₁₀)-alkyl, in which the fragments (C₁-C₁₀)-alkyl are optionally substituted by at least one group selected from NR_{Y1}R_{Y2};
R_{Y1} and R_{Y2} are each independently H or (C₁-C₁₀)-alkyl;
X₁ is H, methyl, ethyl, (C₁-C₁₀)-alkyl-R₄, (C₂-C₁₀)-alkenyl, (C₂-C₁₀)-alkenyl-R₄, CONHR₅, (CH₂-CH₂O)ₘ-H with m=2-4, in which the fragments (C₁-C₁₀)-alkyl are optionally substituted by at least one group selected from halogens;
and N-X₁ optionally represents N⁺-O⁻;
R₂ is chosen from H, methyl, ethyl, (C₁-C₁₀)-alkyl-R_{4'}, (C₂-C₁₀)-alkenyl, (C₂-C₁₀)-alkenyl-R_{4'}, CONHR_{5'}, (CH₂-CH₂O)ₘ-H with m=2-4, (C₁-C₁₀)-alkyl substituted by at least one group selected from halogen, -OH and -O-(C₁-C₁₀)-alkyl;
R₄ is OH, O-(C₁-C₁₀)-alkyl, O-(C₅-C₁₀)-aryl, NO₂, CN, (3-12)-membered-heterocycle, O-((CH₂)₂O)ₙ-H with n=1-3, CONR_{c}R_{d}, halogen, COOR_{c}, CF3, or (C₃-C₁₂)-cycloalkyl,
in which (C₅-C₁₀)-aryl and (3-12)-membered-heterocycle are optionally substituted by at least one group selected from NR_{c}R_{d}, ORₑ, (C₁-C₁₀)-alkyl, halogen and oxo (=O);
R₅ is H, or a group chosen among (C₁-C₁₀)-alkyl-R₆, (C₃-C₁₂)-cycloalkyl, (C₂-C₁₀)-alkenyl, and a (3-12)-membered-heteroaryl, said group being optionally substituted by at least one group selected from halogen, CN, NR_{c}R_{d}, NO₂, CONR_{c}R_{d}, COOR_{c}, CF3, ORₑ,
R_{4'} is OH, O-(C₁-C₁₀)-alkyl, O-(C₅-C₁₀)-aryl, NO₂, CN, NR_{c}R_{d}, (3-12)-membered-heterocycle, O-((CH₂)₂O)ₙ-H with n=1-3, CONR_{c}R_{d}, halogen, COOR_{c}, CF3, or (C3-C₁₂)-cycloalkyl, in which (C₅-C₁₀)-aryl and (3-12)-membered-heterocycle are optionally substituted by at least one group selected from NR_{c}R_{d}, OR_{c}, (C₁-C₁₀)-alkyl, halogen and oxo (=O);
R_{5'} is H, or a group chosen among (C₁-C₁₀)-alkyl-R_{6'}, (C₃-C₁₂)-cycloalkyl, (C₂-C₁₀)-alkenyl, benzyl, and, only when R₁ and X₁ are not H, Me, said groups being optionally substituted by at least one group selected from halogen, CN, NR_{c}R_{d}, NO₂, CONR_{c}R_{d}, COOR_{c}, CF₃, ORₑ;
R6 is NR_{c}R_{d}, (3-12)-membered-heteroaryl or (3-12)-membered-heterocycle, in which the fragment (3-12)-membered-heteroaryl or (3-12)-membered-heterocycle is optionally substituted by at least one group selected from NR_{c}R_{d}, ORₑ, (C₁-C₁₀)-alkyl, (C₁-C₁₀)-alkyl-(3-12)-membered-heterocycle, halogen and oxo;
R_{6'} is NR_{c}R_{d} or (3-12)-membered-heterocycle, in which the (3-12)-membered-heterocycle is optionally substituted by at least one group selected from (C₁-C₁₀)-alkyl;
R₁ is H, methyl, -(C₁-C₆) alkyl-NRaRb, COR₇, (3-12)-membered-azaheterocycle optionally substituted by at least one group selected from (C₁-C₁₀)-alkyl, or (C₁-C₆)-alkyl substituted by at least one group selected from epoxide and (3-12)-membered-azaheterocycle, both optionally substituted by at least one group selected from (C₁-C₁₀)-alkyl,
Ra and R_{b} are independently from each other (C₁-C₆) alkyl or H, at least one of Ra and R_{b} being a (C₁-C₆) alkyl;
R_{c}, Rd and Rₑ are for each occurence independently from each other (C₁-C₆) alkyl or H;
R3 is H, (C₁-C₆) alkyl, (C₁-C₆) alkyl substituted by NR_{c}R_{d};
R₇ is (C₁-C₁₀)-alkyl, (C₅-C₁₀)-aryl;
With the proviso that:
- R₃ is H, and R₂ is H; or
- At least one of Y₁ and Y₂ is not H; or
- At least one of R₁, R₂, R₃ and X₁ is not H or methyl, with R₁, R₂, R₃ and X₁ being such as:
X₁ is H, methyl, CONHR₅, (CH₂-CH₂O)ₘ-H with m=2-4, in which the fragments (C₁-C₁₀)-alkyl are optionally substituted by at least one group selected from halogens;
R2 is chosen from H, methyl, (C₁-C₁₀)-alkyl-R_{4'}, CONHR_{5'}, (CH₂-CH₂O)ₘ-H with m=2-4, (C₁-C₁₀)-alkyl substituted by at least one or two groups selected from halogen, -OH and -O-(C₁-C₁₀)-alkyl, in particular -OH;
R₅ is a group chosen among (C₁-C₁₀)-alkyl-R₆, and a (3-12)-membered-heteroaryl, in particular pyridyl, said group being optionally substituted by at least one group selected from halogen, CN, NR_{c}R_{d}, NO₂, CONR_{c}R_{d}, COOR_{c}, CF3, ORₑ,
R_{4'} is NR_{c}R_{d};
R_{5'} is H, or a group chosen among (C₁-C₁₀)-alkyl-R_{6'} and benzyl, said groups being optionally substituted by at least one group selected from halogen, CN, NR_{c}R_{d}, NO₂, CONR_{c}R_{d}, COOR_{c}, CF₃, ORₑ;
R₆ is (3-12)-membered-heteroaryl, in particular pyridyl, or (3-12)-membered-heterocycle, in particular piperazinyl, in which the fragment (3-12)-membered-heteroaryl or (3-12)-membered-heterocycle is optionally substituted by at least one group selected from NR_{c}R_{d}, ORₑ, (C₁-C₁₀)-alkyl, (C₁-C₁₀)-alkyl-(3-12)-membered-heterocycle, halogen and oxo;
R_{6'} is (3-12)-membered-heterocycle, in which the (3-12)-membered-heterocycle is optionally substituted by at least one group selected from (C₁-C₁₀)-alkyl;
R₁ is H, methyl, -(C₁-C₆)alkyl-NRₐR_{b}, COR₇, (3-12)-membered-azaheterocycle, in particular azepanyl, optionally substituted by at least one group selected from (C₁-C₁₀)-alkyl, or (C₁-C₆)-alkyl substituted by at least one group selected from epoxide and (3-12)-membered-azaheterocycle, in particular pyrrolidinyl or piperazinyl, both epoxide and (3-12)-membered-azaheterocycle being optionally substituted by at least one group selected from (C₁-C₁₀)-alkyl,
Ra and R_{b} are independently from each other (C₁-C₆) alkyl or H, at least one of Ra and R_{b} being a (C₁-C₆) alkyl;
R_{c}, Rd and Rₑ are for each occurence independently from each other (C₁-C₆) alkyl or H;
R3 is H, (C₁-C₆) alkyl, in particular methyl;
R₇ is methyl or phenyl, in particular methyl, or
a stereoisomeric form, a mixture of stereoisomeric forms or a pharmaceutically acceptable salt form thereof,
in admixture with at least one pharmaceutically acceptable excipient.

14. Compound of the following formula (III): 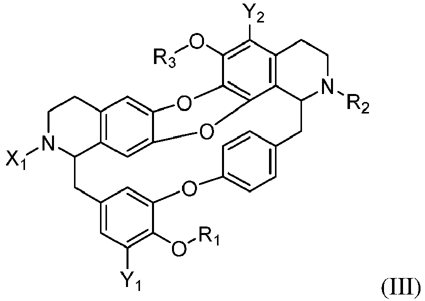 Wherein,
Y₁ and Y₂ are each independently H or (C₁-C₁₀)-alkyl, in which the fragments (C₁-C₁₀)-alkyl are optionally substituted by at least one group selected from NR_{Y1}R_{Y2};
R_{Y1} and R_{Y2} are each independently H or (C₁-C₁₀)-alkyl;
X₁ is H, methyl, ethyl, (C₁-C₁₀)-alkyl-R₄, (C₂-C₁₀)-alkenyl, (C₂-C₁₀)-alkenyl-R₄, CONHR₅, (CH₂-CH₂O)ₘ-H with m=2-4, in which the fragments (C₁-C₁₀)-alkyl are optionally substituted by at least one group selected from halogens;
and N-X₁ optionally represents N⁺-O⁻;
R₂ is chosen from H, methyl, ethyl, (C₁-C₁₀)-alkyl-R_{4'}, (C₂-C₁₀)-alkenyl, (C₂-C₁₀)-alkenyl-R_{4'}, CONHR_{5'}, (CH₂-CH₂O)ₘ-H with m=2-4, (C₁-C₁₀)-alkyl substituted by at least one group selected from halogen, -OH and -O-(C₁-C₁₀)-alkyl;
R₄ is OH, O-(C₁-C₁₀)-alkyl, O-(C₅-C₁₀)-aryl, NO₂, CN, (3-12)-membered-heterocycle, O-((CH₂)₂O)ₙ-H with n=1-3, CONR_{c}R_{d}, halogen, COOR_{c}, CF3, or (C₃-C₁₂)-cycloalkyl, in which (C₅-C₁₀)-aryl and (3-12)-membered-heterocycle are optionally substituted by at least one group selected from NR_{c}R_{d}, ORₑ, (C₁-C₁₀)-alkyl, halogen and oxo (=O);
R₅ is H, or a group chosen among (C₁-C₁₀)-alkyl-R₆, (C₃-C₁₂)-cycloalkyl, (C₂-C₁₀)-alkenyl, and a (3-12)-membered-heteroaryl, said group being optionally substituted by at least one group selected from halogen, CN, NR_{c}R_{d}, NO₂, CONR_{c}R_{d}, COOR_{c}, CF3, ORₑ,
R_{4'} is OH, O-(C₁-C₁₀)-alkyl, O-(C₅-C₁₀)-aryl, NO₂, CN, NR_{c}R_{d}, (3-12)-membered-heterocycle, O-((CH₂)₂O)ₙ-H with n=1-3, CONR_{c}R_{d}, halogen, COOR_{c}, CF3, or (C3-C₁₂)-cycloalkyl, in which (C₅-C₁₀)-aryl and (3-12)-membered-heterocycle are optionally substituted by at least one group selected from NR_{c}R_{d}, OR_{c}, (C₁-C₁₀)-alkyl, halogen and oxo (=O);
R_{5'} is H, or a group chosen among (C₁-C₁₀)-alkyl-R_{6'}, (C₃-C₁₂)-cycloalkyl, (C₂-C₁₀)-alkenyl, benzyl, and, only when R₁ and X₁ are not H, Me, said groups being optionally substituted by at least one group selected from halogen, CN, NR_{c}R_{d}, NO₂, CONR_{c}R_{d}, COOR_{c}, CF₃, ORₑ;
R6 is NR_{c}R_{d}, (3-12)-membered-heteroaryl or (3-12)-membered-heterocycle, in which the fragment (3-12)-membered-heteroaryl or (3-12)-membered-heterocycle is optionally substituted by at least one group selected from NR_{c}R_{d}, ORₑ, (C₁-C₁₀)-alkyl, (C₁-C₁₀)-alkyl-(3-12)-membered-heterocycle, halogen and oxo;
R_{6'} is NR_{c}R_{d} or (3-12)-membered-heterocycle, in which the (3-12)-membered-heterocycle is optionally substituted by at least one group selected from (C₁-C₁₀)-alkyl; R₁ is H, methyl, -(C₁-C₆) alkyl-NRaRb, COR₇, (3-12)-membered-azaheterocycle optionally substituted by at least one group selected from (C₁-C₁₀)-alkyl, or (C₁-C₆)-alkyl substituted by at least one group selected from epoxide and (3-12)-membered-azaheterocycle, both optionally substituted by at least one group selected from (C₁-C₁₀)-alkyl,
Ra and R_{b} are independently from each other (C₁-C₆) alkyl or H, at least one of Ra and R_{b} being a (C₁-C₆) alkyl;
R_{c}, Rd and Rₑ are for each occurence independently from each other (C₁-C₆) alkyl or H;
R3 is H, (C₁-C₆) alkyl, (C₁-C₆) alkyl substituted by NR_{c}R_{d};
R₇ is (C₁-C₁₀)-alkyl, (C₅-C₁₀)-aryl;
With the proviso that:
- R₃ is H, and R₂ is H; or
- At least one of Y₁ and Y₂ is not H; or
- At least one of R₁, R₂, R₃ and X₁ is not H or methyl, with R₁, R₂, R₃ and X₁ being such as:
X₁ is H, methyl, CONHR₅, (CH₂-CH₂O)ₘ-H with m=2-4, in which the fragments (C₁-C₁₀)-alkyl are optionally substituted by at least one group selected from halogens;
R2 is chosen from H, methyl, (C₁-C₁₀)-alkyl-R_{4'}, CONHR_{5'}, (CH₂-CH₂O)ₘ-H with m=2-4, (C₁-C₁₀)-alkyl substituted by at least one or two groups selected from halogen, -OH and -O-(C₁-C₁₀)-alkyl, in particular -OH;
R₅ is a group chosen among (C₁-C₁₀)-alkyl-R₆, and a (3-12)-membered-heteroaryl, in particular pyridyl, said group being optionally substituted by at least one group selected from halogen, CN, NR_{c}R_{d}, NO₂, CONR_{c}R_{d}, COOR_{c}, CF₃, ORe,
R_{4'} is NR_{c}R_{d};
R_{5'} is H, or a group chosen among (C₁-C₁₀)-alkyl-R_{6'} and benzyl, said groups being optionally substituted by at least one group selected from halogen, CN, NR_{c}R_{d}, NO₂, CONR_{c}R_{d}, COOR_{c}, CF₃, ORₑ;
R₆ is (3-12)-membered-heteroaryl, in particular pyridyl, or (3-12)-membered-heterocycle, in particular piperazinyl, in which the fragment (3-12)-membered-heteroaryl or (3-12)-membered-heterocycle is optionally substituted by at least one group selected from NR_{c}R_{d}, ORₑ, (C₁-C₁₀)-alkyl, (C₁-C₁₀)-alkyl-(3-12)-membered-heterocycle, halogen and oxo;
R_{6'} is (3-12)-membered-heterocycle, in which the (3-12)-membered-heterocycle is optionally substituted by at least one group selected from (C₁-C₁₀)-alkyl;
R₁ is H, methyl, -(C₁-C₆)alkyl-NRₐR_{b}, COR₇, (3-12)-membered-azaheterocycle, in particular azepanyl, optionally substituted by at least one group selected from (C₁-C₁₀)-alkyl, or (C₁-C₆)-alkyl substituted by at least one group selected from epoxide and (3-12)-membered-azaheterocycle, in particular pyrrolidinyl or piperazinyl, both epoxide and (3-12)-membered-azaheterocycle being optionally substituted by at least one group selected from (C₁-C₁₀)-alkyl,
Ra and R_{b} are independently from each other (C₁-C₆) alkyl or H, at least one of Ra and R_{b} being a (C₁-C₆) alkyl;
R_{c}, Rd and Rₑ are for each occurence independently from each other (C₁-C₆) alkyl or H;
R3 is H, (C₁-C₆) alkyl, in particular methyl;
R₇ is methyl or phenyl, in particular methyl.
